# EUROPEAN PATENT SPECIFICATION

(11) **EP 4 518 841 B1**
(45) Date of publication and mention of the grant of the patent: **17.12.2025**
(21) Application number: 23725611.0
(22) Date of filing: 05.05.2023
(51) Int. Cl.: A61K 9/28, A61K 9/48

(54) **DOSAGE FORM WITH DRUG RELEASE AT PH 3 TO 6 USING DOUBLE COATING SYSTEM WITH AT LEAST ONE RELEASE ACCELERATION AGENT**
DARREICHUNGSFORM MIT ARZNEIMITTELFREISETZUNG BEI EINEM PH-WERT 3 BIS 5,5 UNTER VERWENDUNG EINES DOPPELBESCHICHTUNGSSYSTEMS MIT MINDESTENS EINEM FREISETZUNGSBESCHLEUNIGUNGSMITTEL
FORME GALÉNIQUE AVEC LIBÉRATION DE MÉDICAMENT AU PH 3 À 5,5 UTILISANT UN SYSTÈME DE DOUBLE ENROBAGE AVEC AU MOINS UN AGENT ACCÉLÉRANT LA LIBÉRATION

(30) Priority: 06.05.2022 US 202263364312 P; 11.05.2022 EP 22172709
(43) Date of publication of application: 12.03.2025
(73) Proprietor: Evonik Operations GmbH, 45128 Essen (DE)
(72) Inventor: SADDIK, Joseph S., Quaker Hill, CT 06375 (US); GOYANI, Chetan, Edison, NJ 08817 (US); PUNNOOSE, Santhosh, New York, NY 10956 (US); SYED, Mohammed Irfan, Hillsborough, NJ 08844 (US); OZA, Kamlesh, Newtown Square, PA 19073 (US); BÄR, Hans, 64720 Michelstadt (DE)
(74) Representative: Evonik Patent Association
(86) International application number: PCT/EP2023/061946
(87) International publication number: WO 2023/214016

(56) References cited:
- WO-A1-2021/115648
- WO-A2-2004/098594
- US-A1- 2003 220 351
- US-A1- 2014 314 846

## Description

### Field of the invention

The invention refers to a dosage form comprising a core comprising at least one biologically active ingredient a specific intermediate coating layer inter alia comprising at least one alkaline agent and at least one release acceleration agent selected from iron oxide, aluminium oxide, titanium dioxide, dimethyl sulfoxide, zinc oxide, sucrose, maltose, lactose, dextrates, glucose, fructose, dyes, or any mixture thereof and an enteric coating layer and a method for obtaining the dosage form. Furthermore, the invention refers to the use of the dosage form according to the present invention for providing at least 80% drug release at pH value 3 to 6 within 30 min.

### Background

A favorable location for release and absorption in the gastro-intestinal tract is the duodenum. However, known dosage forms, for example described in EP application No. 22170267.3 show a lag-time of at least 45 minutes in phosphate buffer pH 5.5 and thus only certain release. Therefore, such a formulation most likely would pass the approximately 25 cm of the Duodenum and enter next the Jejunum without any drug release. Furthermore, such a formulation will not release biologically active ingredients as the trigger pH relevant for the duodenum is often not achieved.

A release of biologically active ingredient in the Duodenum is important for, e.g., Duodenal Ulcer but also systemic drug absorption as for example the mucus layer composition changes and thickness increases.

Therefore, there is a need for enteric dosage forms which show accelerated release especially in the pH range between pH 3 to pH 6, preferably between pH 5 to 6. The desired enteric dosage form shall be enteric with a drug release of not more than 10% after 2 hours in 0.1 N hydrochloric acid (HCl) according to a testing of delayed-release formulations as described in United States Pharmacopeia (USP) 43 monograph 711. After media change from 0.1 N HCl to or direct test without preconditioning in a buffer media with a relevant pH value between pH 3 and 6, preferably between pH 5 to 6 the drug release shall be above 80% within 30 minutes.

It has been surprisingly found by the inventors of the present invention that the addition of certain specific release acceleration agents selected from iron oxide, aluminium oxide, titanium dioxide, dimethyl sulfoxide, zinc oxide, sucrose, maltose, lactose, dextrates, glucose, fructose, dyes, or any mixture thereof in an amount of 0.1 to 20 wt.-% based on the weight of the at least one polymer in the intermediate coating layer leads to an accelerated release at pH values 3 to 6, preferably between pH 5 to 6 within 30 minutes.

### Summary of the invention

The invention refers to a dosage form as set forth in the appended claims comprising
a) a core comprising at least one biologically active ingredient,
b) an intermediate coating layer (ICL) onto or above the core, comprising
   i) at least one polymer;
   ii) at least one alkaline agent;
   iii) at least one release acceleration agent selected from iron oxide, aluminium oxide, titanium dioxide, dimethyl sulfoxide, zinc oxide, sucrose, maltose, lactose, dextrates, glucose, fructose, dyes, or any mixture thereof;
   iv) optionally at least one glidant
   v) optionally at least one plasticizer; and
   vi) optionally at least one additive, different from i) to v); wherein
   the at least one release acceleration agent is present in 0.1 to 20 wt.-%, preferably 0.2 to 15 wt.-%, more preferably 0.5 to 8 wt.-%, most preferably 1 to 6 wt.-%, based on the weight of the at least one polymer; and
c) an enteric coating layer (ECL) onto or above the intermediate coating layer, comprising
   i) at least one polymer;
   ii) optionally at least one glidant;
   iii) optionally at least one emulsifier;
   iv) optionally at least one plasticizer;
   v) optionally at least one biologically active ingredient; and
   vi) optionally at least one additive, different from i) to v).

In one embodiment the at least one polymer of the enteric coating layer comprises or consist of two different polymers, preferably wherein the first polymer is an anionic polymer having a Tgm ≥ 35°C, more preferably 35 to 155 °C, even more preferred 80 to 145 °C, most preferably 90 to 125 °C; and wherein the second polymer is preferably a polymer having a Tgm of ≤ 30 °C, more preferably ≤ 15 °C.

In one embodiment the total coating amount of the coating layers is 2.0 to 30 mg/cm², preferably 2.0 to 10 mg/cm² or 10 to 25 mg/cm².

In a second aspect the invention pertains to a method of obtaining the dosage form according to the present invention, wherein the intermediate coating layer is coated on the core via spray coating and thereafter the enteric coating layer is coated on the intermediate coating layer via spray coating.

In a third aspect the invention refers to the use of the polymer-coated hard shell capsule according to the present invention for delayed release, i.e. for providing at least 80% drug release at pH value 3 to 6, preferably between pH 5 to 6 within 30 min.

### Detailed description of the invention

### Polymer or polymer mixture comprised in the intermediate or enteric coating layer

In the following polymers, which are suitable for being used as the at least one polymer in the intermediate or one of the at least one polymer in the enteric coating layer, are disclosed. The at least one polymer in the intermediate coating layer as well as in the enteric coating layer can be any of the below-mentioned polymers. The enteric coating layer preferably requires a first polymer, wherein the first polymer is an anionic polymer having a Tgm ≥ 35°C, preferably 35 to 155 °C, more preferably 80 to 145 °C, most preferably 90 to 125 °C; and a second polymer, wherein the second polymer is a polymer having a Tgm of ≤ 30 °C, preferably ≤ 20 °C, more preferred -10 to 20 °C. Suitable anionic and neutral polymers are described in the following as well. If applicable to all polymers of both coating layers the expression "polymer comprised in / of the coating layer" or the like is used.

The polymer comprised in the coating layer is preferably a film-forming polymer.

The at least one polymer of the coating layer can be selected from the group of anionic polymers, cationic polymers and neutral polymers or any mixture thereof.

The selection of generic or specific polymer features or embodiments as disclosed herein can be combined without restriction with any other generic or specific selection of material or numerical features or embodiments as disclosed herein, such as capsule materials, capsule sizes, coating thicknesses, biologically active ingredients and any other features or embodiments as disclosed.

The coating layer, which can be a single layer or can comprise or consist of two or more individual layers, can comprise in total 10 to 100, 20 to 95, 30 to 90 % by weight of one or more polymers, preferably (meth)acrylate copolymer(s).

The proportions of monomers mentioned for the respective polymers in general add up to 100% by weight.

The intermediate coating layer and the enteric coating layer are different from each other.

In a preferred embodiment a further coating layer, a top coating layer is contained. The top coating layer comprises at least one cationic polymer or at least one neutral polymer or any mixture thereof. In a preferred embodiment the top coating layer is selected from at least one natural polymer or a starch, preferably as described below or hydroxypropyl methylcellulose, most preferred is hydroxypropyl methylcellulose.

### Glass transition temperature Tgm

The glass transition temperature Tgm according to the present invention is preferably determined by Differential Scanning Calorimetry (DSC) according to ISO 11357-2:2013-05. The determination is performed with a heating rate of 20 K/min. The glass transition temperature Tgm can as well be determined by half step height method as described in section 10.1.2 of DIN EN ISO 11357-2.

### Anionic polymers

The at least one polymer comprised in the coating layer can be an anionic polymer selected from the group of anionic (meth)acrylate copolymers, anionic polyvinyl polymers or copolymers and anionic celluloses.

### Anionic (meth)acrylate copolymers

Preferably the anionic (meth)acrylate copolymer comprises 25 to 95, preferably 40 to 95, in particular 60 to 40, % by weight free-radical polymerized C1- to C12-alkyl esters, preferably C1- to C4-alkyl esters of acrylic or of methacrylic acid and 75 to 5, preferably 60 to 5, in particular 40 to 60 % by weight (meth)acrylate monomers having an anionic group. The proportions mentioned in general add up to 100% by weight. However, it is also possible in addition, without this leading to an impairment or alteration of the essential properties, for small amounts in the region of 0 to 10, for example 1 to 5, % by weight of further monomers capable of vinylic copolymerization, such as, for example, hydroxyethyl methacrylate or hydroxy-ethyl acrylate, to be present. It is preferred that no further monomers capable of vinylic copolymerization are present.

C1- to C4-alkyl esters of acrylic or methacrylic acid are in particular methyl methacrylate, ethyl methacrylate, butyl methacrylate, methyl acrylate, ethyl acrylate and butyl acrylate.

A (meth)acrylate monomer having an anionic group is, for example, acrylic acid, with preference for methacrylic acid.

Suitable anionic (meth)acrylate copolymers are those polymerized from of 40 to 60% by weight methacrylic acid and 60 to 40% by weight methyl methacrylate or 60 to 40% by weight ethyl acrylate (EUDRAGIT^{®} L or EUDRAGIT^{®} L 100 55 types).

EUDRAGIT^{®} L is a copolymer polymerized from 50% by weight methyl methacrylate and 50% by weight methacrylic acid. The pH value of the start of the specific active ingredient release in intestinal juice or simulated intestinal fluid can be stated to be at about pH value 6.0. It has a Tgm of > 125°C.

EUDRAGIT^{®} L 100-55 is a copolymer polymerized from 50% by weight ethyl acrylate and 50% by weight methacrylic acid. EUDRAGIT^{®} L 30 D-55 is a dispersion comprising 30% by weight EUDRAGIT^{®} L 100-55.

Likewise, suitable are anionic (meth)acrylate copolymers polymerized from 20 to 40% by weight methacrylic acid and 80 to 60% by weight methyl methacrylate (EUDRAGIT^{®} S type). The pH value of the start of the specific active ingredient release in intestinal juice or simulated intestinal fluid can be stated to be at about pH value 7.0.

Suitable (meth)acrylate copolymers are polymerized from 10 to 30% by weight methyl methacrylate, 50 to 70% by weight methyl acrylate and 5 to 15% by weight methacrylic acid (EUDRAGIT^{®} FS type).

EUDRAGIT^{®} FS is a copolymer polymerized from 25% by weight methyl methacrylate, 65% by weight methyl acrylate and 10% by weight methacrylic acid. EUDRAGIT^{®} FS 30 D is a dispersion comprising 30% by weight EUDRAGIT^{®} FS.

Suitable is a copolymer composed of
20 to 34% by weight methacrylic acid and/or acrylic acid,
20 to 69% by weight methyl acrylate and
0 to 40% by weight ethyl acrylate and/or where appropriate
0 to 10% by weight further monomers capable of vinylic copolymerization,
with the proviso that the glass transition temperature of the copolymer according to ISO 11357-2:2013-05, subsection 3.3.3, is not more than 60°C.

Suitable is a copolymer polymerized from
20 to 33% by weight methacrylic acid and/or acrylic acid,
   5 to 30% by weight methyl acrylate and
   20 to 40% by weight ethyl acrylate and
   more than 10 to 30% by weight butyl methacrylate and where appropriate
   0 to 10% by weight further monomers capable of vinylic copolymerization,
   where the proportions of the monomers add up to 100% by weight,
with the proviso that the glass transition temperature of the copolymer according to ISO 11357-2:2013-05, subsection 3.3.3 (midpoint temperature Tm,g), is 55 to 70°C.

The copolymer preferably consists of 90, 95 or 99 to 100% by weight of the monomers methacrylic acid, methyl acrylate, ethyl acrylate and butyl methacrylate in the ranges of amounts indicated above. However, it is possible, without this necessarily leading to an impairment of the essential properties, for small amounts in the range from 0 to 10, e.g., 1 to 5% by weight of further monomers capable of vinylic copolymerization additionally to be present, such as, for example, methyl methacrylate, butyl acrylate, hydroxyethyl methacrylate, vinylpyrrolidone, vinyl-malonic acid, styrene, vinyl alcohol, vinyl acetate and/or derivatives thereof.

Further suitable anionic (meth)acrylate copolymers can be so called core/shell polymers as described in WO 2012/171575 A2 or WO 2012/171576 A1. A suitable Core Shell polymer is a copolymer from a two-stage emulsion polymerization process with a core of 75 % by weight comprising polymerized units of 30% by weight of ethyl acrylate and 70% by weight of methyl methacrylate and a shell of polymerized units comprising 25 % by weight of polymerized from 50% by weight ethyl acrylate and 50% by weight methacrylic acid.

A suitable Core-Shell polymer can be a copolymer from a two-stage emulsion polymerization process with a core with 70 to 80 % by weight, comprising polymerized units of 65 to 75 % by weight of ethyl acrylate and 25 to 35 % by weight of methyl methacrylate, and a shell with 20 to 30 % by weight, comprising polymerized units of 45 to 55 % by weight ethyl acrylate and 45 to 55 % by weight methacrylic acid.

### Anionic celluloses

Anionic celluloses can be selected from carboxymethyl ethyl cellulose and its salts, cellulose acetate phthalate (CAP), cellulose acetate succinate (CAS), cellulose acetate trimellitate (CAT), hydroxypropyl methyl cellulose phthalate (HPMCP, HP50, HP55), hydroxypropyl methyl cellulose acetate succinate (HPMCAS-LF, -MF, -HF).

The coating layer can comprise one or more anionic cellulose(s), ethyl cellulose and/or one or more starches comprising at least 35 % by weight amylose, preferably with a glass transition temperature Tgm of 130 °C or less (determined by Differential Scanning Calorimetry (DSC) according to ISO 11357-2:2013-05), wherein the coating layer is preferably present in an amount of about 1 to 5.8, more preferably 2 to 5 mg/cm².

The coating layer can comprise in total 10 to 100, 20 to 95, 30 to 90 % by weight of one or more anionic cellulose(s), ethyl cellulose and/or one or more starches comprising at least 35 % by weight amylose.

The glass transition temperature Tgm of hydroxypropyl methyl cellulose phthalate is about 132 to 138 °C (type HP-55 about 133 °C, type HP-50 about 137 °C).

The glass transition temperature Tgm of hydroxypropyl methyl cellulose acetate succinate (HPMCAS) is about 120 °C (AquaSolveTM L HPMCAS 119°C, AquaSolveTM M HPMCAS 120°C, AquaSolveTM H HPMCAS 122°C).

### Anionic vinyl copolymers

Anionic vinyl copolymers can be selected from unsaturated carboxylic acids other than acrylic acid or methacrylic acid as exemplified by polyvinylacetatephthalate or a copolymer of vinylacetate and crotonic acid (preferably at a ratio of 9:1).

### Cationic polymers

A suitable cationic (meth)acrylate copolymer comprised in the coating layer can be polymerized from monomers comprising C1- to C4-alkyl esters of acrylic or of methacrylic acid and an alkyl ester of acrylic or of methacrylic acid with a tertiary or a quaternary ammonium group in the alkyl group. The cationic, water-soluble (meth)acrylate copolymer can be polymerized partly or fully of alkyl from acrylates and/or alkyl methacrylates having a tertiary amino group in the alkyl radical. A coating comprising these kinds of polymers may have the advantage of providing moisture protection to the hard shell capsule. Moisture protection shall be understood a reduced uptake of moisture or water during storage of the readily filled and final-locked capsules.

A suitable cationic (meth)acrylate copolymer can be polymerized from 30 to 80% by weight of C1-to C4-alkyl esters of acrylic or of methacrylic acid, and 70 to 20% by weight of alkyl(meth)acrylate monomers having a tertiary amino group in the alkyl radical.

The preferred cationic (meth)acrylate copolymer can be polymerized from 20 - 30% by weight of methyl methacrylate, 20 - 30% by weight of butyl methacrylate and 60 - 40% by weight of dimethylaminoethyl methacrylate (EUDRAGIT^{®} E type polymer).

A specifically suitable commercial (meth)acrylate copolymer with tertiary amino groups is polymerized from 25% by weight of methyl methacrylate, 25% by weight of butyl methacrylate and 50% by weight of dimethylaminoethyl methacrylate (EUDRAGIT^{®} E 100 or EUDRAGIT^{®} E PO (powder form)). EUDRAGIT^{®} E 100 and EUDRAGIT^{®} E PO are water-soluble below approx. pH value 5.0 and are thus also gastric juice-soluble.

A suitable (meth)acrylate copolymer can be composed of 85 to 98% by weight of free-radical polymerized C1 to C4 alkyl esters of acrylic or methacrylic acid and 15 to 2% by weight of (meth)acrylate monomers with a quaternary amino group in the alkyl radical.

Preferred C1 to C4 alkyl esters of acrylic or methacrylic acid are methyl acrylate, ethyl acrylate, butyl acrylate, butyl methacrylate and methyl methacrylate.

Further suitable cationic (meth)acrylate polymers may contain polymerized monomer units of 2-trimethylammonium-ethyl methacrylate chloride or trimethylammonium-propyl methacrylate chloride.

An appropriate copolymer can be polymerized from 50 to 70% by weight of methyl methacrylate, 20 to 40% by weight of ethyl acrylate and 7 to 2% by weight of 2-trimethylammoniumethyl methacrylate chloride.

A specifically suitable copolymer is polymerized from 65% by weight of methyl methacrylate, 30% by weight of ethyl acrylate and 5% by weight of 2-trimethylammoniumethyl methacrylate chloride (EUDRAGIT^{®} RS).

A further suitable (meth)acrylate copolymer can be polymerized from 85 to less than 93% by weight of C1 to C4 alkyl esters of acrylic or methacrylic acid and more than 7 to 15% by weight of (meth)acrylate monomers with a quaternary amino group in the alkyl radical. Such (meth)acrylate monomers are commercially available and have long been used for release-slowing coatings.

A specifically suitable copolymer is polymerized from 60% by weight of methyl methacrylate, 30% by weight of ethyl acrylate and 10% by weight of 2-trimethylammoniumethyl methacrylate chloride (EUDRAGIT^{®} RL).

### Neutral polymers

Neutral polymers are defined as polymers which are polymerized from neutral monomers and less than 5, preferably less than 2 % by weight or most preferred no monomers with ionic groups.

Suitable neutral polymers for the coating of the hard shell capsule are methacrylate copolymers, preferably copolymers of ethyl acrylate and methyl methacrylate like EUDRAGIT^{®} NE or EUDRAGIT^{®} NM, neutral celluloses, such as methyl-, ethyl- or propyl ethers of cellulose, for instance hydroxypropyl cellulose, polyvinyl pyrrolidone, polyvinyl acetate or polyvinyl alcohol.

Neutral methacrylate copolymers are often useful in mixture with anionic (meth)acrylate copolymers.

Neutral methacrylate copolymers are polymerized from at least to an extent of more than 95% by weight, in particular to an extent of at least 98% by weight, preferably to an extent of at least 99% by weight, in particular to an extent of at least 99% by weight, more preferably to an extent of 100% by weight, of (meth)acrylate monomers with neutral radicals, especially C1- to C4-alkyl radicals.

Suitable (meth)acrylate monomers with neutral radicals are, for example, methyl methacrylate, ethyl methacrylate, butyl methacrylate, methyl acrylate, ethyl acrylate, butyl acrylate. Preference is given to methyl methacrylate, ethyl acrylate and methyl acrylate.

Methacrylate monomers with anionic radicals, for example acrylic acid and/or methacrylic acid, can be present in small amounts of less than 5% by weight, preferably not more than 2% by weight, more preferably not more than 1 or 0.05 to 1% by weight.

Suitable examples are neutral or virtually neutral (meth)acrylate copolymers polymerized from 20 to 40% by weight of ethyl acrylate, 60 to 80% by weight of methyl methacrylate and 0 to less than 5% by weight, preferably 0 to 2 or 0.05 to 1% by weight of methacrylic acid or acrylic acid.

Suitable examples are neutral or virtually neutral (meth)acrylate copolymers polymerized from 20 to 40% methyl methacrylate by weight of, 60 to 80% by weight of ethyl acrylate and 0 to less than 5% by weight, preferably 0 to 2 or 0.05 to 1% by weight of methacrylic acid or acrylic acid. (EUDRAGIT^{®} NE or EUDRAGIT^{®} NM type).

EUDRAGIT^{®} NE and EUDRAGIT^{®} NM are copolymers comprising free-radically polymerized units of 28 to 32% by weight of methyl methacrylate and 68 to 72% by weight of ethyl acrylate.

Preference is given to neutral or essentially neutral methyl acrylate copolymers which, according to WO 01/68767 A1, have been prepared as dispersions using 1 - 10% by weight of a non-ionic emulsifier having an HLB value of 15.2 to 17.3. The latter offer the advantage that there is no phase separation with formation of crystal structures by the emulsifier (EUDRAGIT^{®} NM type).

According to EP 1 571 164 A2, corresponding, virtually neutral (meth)acrylate copolymers with small proportions of 0.05 to 1% by weight of monoolefinically unsaturated C3-C8-carboxylic acids can, however, also be prepared by emulsion polymerization in the presence of comparatively small amounts of anionic emulsifiers, for example 0.001 to 1% by weight.

### Natural polymers

Especially for nutraceutical dosage forms so called "natural polymer" coatings are preferred by many customers. Natural polymers are based on a source from nature, plants, microorganisms or animals, but sometimes further chemically processed. Natural polymers for coatings can be selected from polymers such as starch, alginates or salts of alginates, preferably sodium alginate, pectin, shellac, zein, carboxymethyl-zein, modified starch, for instance EUDRAGUARD^{®} Natural, marine sponge collagen, chitosan, gellan gum. Suitable polymer mixtures may comprise:
Ethyl cellulose and pectin, modified starch (EUDRAGUARD^{®} Natural) and alginate and/or pectin, shellac and alginate and/or pectin, shellac and inulin, whey protein and gums (such as guar gum or tragacanth gum), zein and polyethylene glycol, sodium alginate and chitosan.

Ethyl cellulose is a derivative of cellulose in which some of the hydroxyl groups of the repeating glucose units are converted into ethyl ether groups. Ethyl cellulose can be used as a delayed release coating material for the capsules as disclosed. The glass transition temperature Tgm of ethyl cellulose can be in the range of about 128 to 130 °C (Hui Ling Lai et al. Int.J.Pharmaceuticals 386 (2010) 178-184).

In the following further components, which can be present in the enteric coating layer or intermediate coating layer are described.

Unless explicitly state otherwise, the components are in general suitable to be used in both coating layers. The amount of the respective component is indicated in view of the total weight of the at least one polymer, contained in the respective coating layer, unless explicitly stated otherwise.

### Glidants

Glidants usually have lipophilic properties. They prevent agglomeration of cores during film formation of the film forming polymers.

The at least one glidant is preferably selected from silica, for example commercially available under the tradenames RXCIPIENTS^{®} GL100 or RXCIPIENTS^{®} GL200, ground silica, fumed silica, kaolin calcium silicate, magnesium silicate, colloidal silicone dioxide, talc, stearate salts like calcium stearate, magnesium stearate, zinc stearate, sodium stearyl fumarate, starch, stearic acid, preferably talc, magnesium stearate, colloidal silicon dioxide und glycerol monostearate or mixtures thereof, more preferred glycerol monostearate and talc or mixtures thereof.

Standard proportions for use of glidants in the coating layer range between 0.5 and 100 % by weight, preferably 3 to 75 % by weight, more preferably 5 to 50 % by weight, most preferably 5 to 30 % by weight, relative to the total weight of the at least one polymer.

### Emulsifiers

In general, all known emulsifiers are suitable. Preferred are non-ionic emulsifier, in particular emulsifier having an HLB > 10 or HLB > 12. The HBL Value can be determined according to Griffin, William C. (1954), "Calculation of HLB Values of Non-Ionic Surfactants" (PDF), Journal of the Society of Cosmetic Chemists, 5 (4): 249-56.

The at least one emulsifier is preferably selected from polyglycosides, alcohols, sugar and sugar derivatives, polyethers, amines, polyethylene derivatives, alkyl sulfates (e.g., sodium dodecyl sulfate), alkyl ether sulfates, dioctyl sodium sulfosuccinate, polysorbates (e.g. polyoxyethylene (20) sorbitan monooleate), nonylphenol ethoxylates (nonoxynol-9) and mixtures thereof.

The at least one emulsifier is preferably selected from alkyl polyglycosides, decyl glucoside, decyl polyglucose, lauryl glucoside, octyl glucoside, N-octyl beta-D-thioglucopyranoside, cetostearyl alcohol, cetyl alcohol, stearyl alcohol, polyoxyethylene cetostearyl alcohol, cetylstearyl alcohol, oleyl alcohol, polyglyceryl-6-dioleate, glyceryl stearate citrate, polyglyceryl-3 caprate, polyglyceryl-3 diisostearate, glyceryl isostearate, polyglyceryl-4-isostearate, glyceryl monolinoleate, dicaprylyl carbonate, alcohol polyglycol ether, polyethylenglycolether of cetearylalcohols (n=20), polyethylene glycol-6 stearate, glycol stearate, polyethylene glycol-32 stearate, polyethylene glycol-20 stearate, fatty alcohol polyglycol ether, polyethylene glycol-4 laurate, polyethylene glycol isocetyl ether (n=20), polyethyleneglycol-32 (Mw 1500 g/mol) mono- and diesters of lauric acid (C12), nonaethylene glycol, polyethylene glycol nonylphenyl ether, octaethylene glycol monododecyl ether, pentaethylene glycol monododecyl ether, polyethylene glycol macrocetyl ether, polyethylene glycol esters of palmitic (C16) or stearic (C18) or caprylic acids, polyoxyethylene fatty ether derived from stearyl alcohols like BRIJ S2, polyoxyethylene oxypropylene stearate, macrogol stearyl ether (20), diethylaminoethyl stearate, polyethylene glycol stearate, sucrose distearate, sucrose tristearate, sorbitan monostearate, sorbitan tristearate, mannide monooleate, octaglycerol monooleate, sorbitan dioleate, polyricinoleate, polysorbate like polysorbate 20 and Polyoxyethylene (20) sorbitan monooleate (polysorbate 80), sorbitan, sorbitan monolaurate, sucrose cocoate, glycereth-2 cocoate, ethylhexyl cocoate, polypropylene glycol -3 benzyl ether myristate, sodium myristate, gold sodium thiomalate, polyethylene glycol 8 laurate, polyethylene-4 dilaurate, from α-Hexadecyl-ω-hydroxypoly(oxyethylene), cocamide diethanolamine, N-(2-hydroxyethyl)dodecanamide, octylphenoxypolyethoxyethanol, maltoside, 2,3-Dihydroxypropyl dodecanoate, 3-[(3R,6R,9R,12R,15S,22S,25S,30aS)-6,9,15,22-Tetrakis(2-amino-2-oxoethyl)-3-(4-hydroxybenzyl)-12-(hydroxymethyl)-18-(11-methyltridecyl)-1,4,7,10,13,16,20,23,26-nonaoxotriacontahydropyrrolo[1,2-g][1,4,7,10,13,16,19,22,25]nonaazacyclooctacosin-25-yl]propenamide, 2-{2-[2-(2-{2-[2-(2-{2-[2-(4-nonylphenoxy)ethoxy]ethoxy}ethoxy)ethoxy]ethoxy}ethoxy)ethoxy]ethoxy}ethanol, oxypolyethoxydodecane, poloxamers like poloxamer 188 (Pluronic F-68) and poloxamer 407, propylene glycol monocaprylate, type I (Capryol PGMC), polyethoxylated tallow amine, polyglycerol, polyoxyl 40 hydrogenated castor oil, surfactin, 2-[4-(2,4,4-trimethylpentan-2-yl)phenoxy]ethanol, carbomer, sodium carbomer, carboxymethylcellulose calcium, carrageenan, cholesterol, deoxycholic acid, phospholipids like egg phospholipids, gellan gum, lanolin, capric acid, waxes like Polawax NF, Polawax A31 or Ceral PW, ester gum, dea-cetyl phosphate, soya lecithin, sphingomyelins, sodium phosphate, sodium lauroyl lactylate, lanolin, Oxirane methyl-polymer with oxirane monobutyl ether, 1,2-dierucoylphosphatidylcholine, dimethicone end-blocked with an average of 14 moles of propylene oxide, laurylmethicone copolyol, lauroglycol 90, white mineral oil like Amphocerine KS, dispersion of acrylamide/sodium acryloyldimethyl taurate copolymer in isohexadecane, and sodium polyacrylate or mixtures thereof. Preferred are macrogol stearyl ether (20) and polysorbate 80.

In one embodiment less than 3 wt.-%, preferably 1.5 wt.-% of at least one emulsifier based on the total weight of the at least one polymer is present or essentially no or no emulsifier is present.

### Enteric or top coating layer

The enteric or top coating layer may comprise 10 % or more, 20 % or more, 30 % or more, 40 % or more, 50 % or more, 60 % or more, 70 % or more, 80 % or more, 90 % or more by weight or 95 % or more by weight of the polymers. The coating layer may comprise 10 to 100, 10 to 90, 12 to 80, 15 to 80, 18 to 80, 20 to 80 or 40 to 80 % by weight of the polymers.

The top coating layer is located on top of the enteric coating layer, comprising the at least one polymer as described above. A top coat is also preferably water-soluble or essentially water-soluble. A top coat may have the function of colouring the pharmaceutical or nutraceutical form or protecting from environmental influences for instance from moisture during storage.

### Intermediate coating layer

The intermediate coating layer is located upon or above the capsule and comprises at least one polymer, at least one alkaline agent, and at least one specific release acceleration agent.

The intermediate coating layer can comprise 5 to 90 % by weight, preferably 10 to 70 % by weight, more preferably 30 to 50 % by weight of the at least one polymer based on the total weight of the intermediate coating layer. The at least one polymer is preferably selected from celluloses, like hydroxyethyl cellulose (HEC), hydroxypropyl cellulose (HPC), hydroxypropyl methyl cellulose (HPMC), hydroxyethyl methyl cellulose (HEMC), ethyl cellulose (EC), methyl cellulose (MC), cellulose esters, cellulose glycolates, polyethylene glycols, polyethylene oxides, polyvinyl pyrrolidone, polyvinyl acetate, polyvinyl alcohol, or a mixture thereof, preferably is hydroxypropyl methyl cellulose.

The intermediate coating layer can comprise 10 to 75, preferably 10 to 50, % by weight of the alkaline agent, based on the total weight of the coating layer.

The alkaline agent can be an alkali or an earth alkali metal salt. The alkaline agent can be selected from calcium oxide, calcium carbonate, magnesium carbonate, magnesium oxide, sodium carbonate, sodium bicarbonate and sodium hydroxide or any mixtures thereof. Preferred alkaline agents are magnesium oxide and/or magnesium carbonate.

The at least one release acceleration agent is selected from iron oxide, aluminium oxide, titanium dioxide, dimethyl sulfoxide, zinc oxide, sucrose, maltose, lactose, dextrates, glucose, fructose, dyes like Red 40 (E129), Blue 1 (E133), and Yellow 5 (E102), Amaranth (E123), Carmoisine (E122), Ponceau 4R (E124) and Quinoline Yellow WS (E104), Brilliant Blue (E133), Indigo Carmine (E132), Allura Red (E129), Tartrazine (E102), Sunset Yellow (E110), Erythrosine (E127), Ponceau 4R (E124), or any mixture thereof and is present in 0.1 to 20 wt.-%, preferably 0.2 to 15 wt.-%, more preferably 0.5 to 8 wt.-%, most preferably 1 to 6 wt.-%, based on the weight of the at least one polymer.

The intermediate coating layer can comprise at least one glidant, preferably in an amount of 2 to 50, more preferably 5 to 25 % by weight, based on the total weight of the intermediate coating layer. Suitable glidants are the same as described for the enteric coating layer herein. The at least one glidant is preferably talc or glycerol.

The intermediate layer can comprise 1 to 95, preferably 10 to 50 % by weight of at least one additive based on the total weight of the intermediate layer, such as a polymeric binder, a plasticizer or an anti-tacking agent or a combination thereof. Suitable additives are the same as described for the enteric coating layer.

Preferably the intermediate layer is directly located upon the capsule.

### Amount and thickness of the enteric and intermediate coating layer

In order to ensure influx prevention and processability, when a hard shell capsule, tablet or mini-tablet forms the core, it has been found that the total coating amount is preferably 2.0 to 30 mg/cm², more preferably 2.0 to 10 mg/cm² in particular for hard shell capsules or 10 to 25 mg/cm² in particular for mini-tablet or tablets.

For a hard shell capsule of size #0, the amount of the coating layer should not be too high. If the amount of coating layer applied is too high this may result in difficulties to process the polymer-coated pre-locked hard shell capsules subsequently in a capsule-filling machine. If the amount of coating layer is less than 5 mg/cm2, for instance 2 to 4 mg/cm2 usually no problem with standard capsule-filling machines without modification will occur. In the range from 4 and up to about 8 mg/cm2 capsule-filling machines can still be used, however the forms for the bodies and the caps should be adjusted to be somewhat wider. Such an adjustment can be easily performed by a mechanical engineer. Thus capsule-filling machines can be advantageously used within a range of an amount of coating layer from about 3 to about 8 mg/cm².

For a hard shell capsule of size #1, the amount of the coating layer should not be too high. If the amount of coating layer applied is too high this may result in difficulties to process the polymer-coated pre-locked hard shell capsules subsequently in a capsule-filling machine. If the amount of coating layer is less than 4 mg/cm2, for instance 2 to 3.5 mg/cm2 usually no problem with standard capsule-filling machines without modification will occur. In the range from 3.5 and up to about 8 mg/cm2 capsule-filling machines can still be used, however the forms for the bodies and the caps should be adjusted to be somewhat wider. Such an adjustment can be easily performed by a mechanical engineer. Thus capsule-filling machines can be advantageously used within a range of an amount of coating layer from about 3 to about 8 mg/cm2.

For a hard shell capsule of size #3, the amount of the coating layer should not be too high. If the amount of coating layer applied is too high this may result in difficulties to process the polymer-coated pre-locked hard shell capsules subsequently in a capsule-filling machine. In the range from 2 and up to about 6 mg/cm2 capsule-filling machines can still be used, however the forms for the bodies and the caps should be adjusted to be somewhat wider. Such an adjustment can be easily performed by a mechanical engineer. Thus capsule-filling machines can be advantageously used within a range of an amount of coating layer from about 3 to about 6 mg/cm2.

If the amount of coating layer applied is too high there will be also an assembly of too much coating layer at the rim of the cap where the gap between body and cap is in the pre-locked state. This may result after drying in fissures of the coating layer when the coated pre-locked hard shell capsule is opened manually or in a machine. The fissures may result in a later leakage of the capsule. Finally, a too thick coating may result in difficulties or make it impossible to close the opened coated hard shell capsule to the final-locked state since the coating layer is thicker than the gap in the overlapping area between the body and the cap.

As a rough rule the coating layer on the hard shell capsule, tablet or mini-tablet can be applied in an amount (= a total weight gain) of 0.7 to 30, 1.0 - 18, 2 to 10, 4 to 8, 1.0 to 8, 1.5 to 5.5, 1.5 to 4 mg/cm².

As a rough rule the coating layer on the hard shell capsule, tablet or mini-tablet may have an average thickness of about 5 to 100, 10 to 50, 15 to 75 µm.

As a rough rule the coating layer on the hard shell capsule can be applied in an amount of 5 to 50, preferably 8 - 40 % dry weight in relation to the weight of the pre-locked capsule.

With this guidance a skilled person will be able to adjust the amounts of the coating layer in a range between too low and too high.

### Biologically active ingredient

The biologically active ingredient is preferably a pharmaceutical active ingredient and/or a nutraceutical active ingredient and/or a cosmetically active ingredient. Even though it is possible that certain biologically active ingredients are contained in the respective coating layers, it is preferred that the biologically active ingredient is contained in the fill. In particular, if the biologically active ingredient is comprised in a liposome, lipid nanoparticle or nucleic acid, the biologically active ingredient is only contained in the fill.

### Biologically active ingredients

The invention is particularly useful for delayed release formulated pharmaceutical or nutraceutical dosage forms with a fill of biologically active, preferably pharmaceutical or nutraceutical active, ingredients.

Suitable therapeutic and chemical classes of pharmaceutical active ingredients which members can be used as fill for the described polymer-coated hard shell capsules are for instance: analgesics, antibiotics or anti-infectives, antibodies, antiepileptics, antigens from plants, antirheumatics, benzimidazole derivatives, beta-blocker, cardiovascular drugs, chemotherapeutics, CNS drugs, digitalis glycosides, gastrointestinal drugs, e.g. proton pump inhibitors, enzymes, hormones, liquid or solid natural extracts, oligonucleotides, peptide, hormones, proteins, therapeutic bacteria, peptides, proteins (metal)salt i.e. aspartates, chlorides, urology drugs, lipid nanoparticles, liposomes, polymeric nanoparticles, vaccines. In a preferred embodiment at least one liposome or lipid nanoparticle each comprising at least one polynucleotide is contained.

In a preferred embodiment the pharmaceutically active ingredient is a lipid nanoparticle or liposome each comprising a polynucleotide or a nucleic acid, more preferably a nucleic acid agent can be DNA, RNA, or combinations thereof. In some embodiments, a nucleic acid agent can be an oligonucleotide and/or polynucleotide. In some embodiments, a nucleic acid agent may be an oligonucleotide and/or modified oligonucleotide (including, but not limited to, modifications through phosphorylation); an antisense oligonucleotide and/or modified antisense oligonucleotide (including, but not limited to, modifications through phosphorylation). In some embodiments, a nucleic acid agent can comprise cDNA and/or genomic DNA. In some embodiments, a nucleic acid agent can comprise non-human DNA and/or RNA (e.g., viral, bacterial, or fungal nucleic acid sequences). In some embodiments, a nucleic acid agent can be a plasmid, cosmid, gene fragment, artificial and/or natural chromosome (e.g., a yeast artificial chromosome), and/or a part thereof. In some embodiments, a nucleic acid agent can be a functional RNA (e.g., mRNA, a tRNA, an rRNA and/or a ribozyme). In some embodiments, a nucleic acid agent can be an RNAi-inducing agent, small interfering RNA (siRNA), short hairpin RNA (shRNA), and/or microRNA (miRNA). In some embodiments, a nucleic acid agent can be a peptide nucleic acid (PNA). In some embodiments, a nucleic acid agent can be a polynucleotide comprising synthetic analogues of nucleic acids, which may be modified or unmodified. In some embodiments, a nucleic acid agent can comprise various structural forms of DNA including single-stranded DNA, double-stranded DNA, supercoiled DNA and/or triple -helical DNA; Z-DNA; and/or combinations thereof. Further suitable nucleic acids are for example disclosed in WO 2012103035 A1, which are incorporated by reference.

Further examples of drugs that can be used as fill for the described polymer-coated hard shell capsules are for instance acamprosat, aescin, amylase, acetylsalicylic acid, adrenalin, 5-amino salicylic acid, aureomycin, bacitracin, balsalazine, beta carotene, bicalutamid, bisacodyl, bromelain, bromelain, budesonide, calcitonin, carbamacipine, carboplatin, cephalosporins, cetrorelix, clarithromycin, chloromycetin, cimetidine, cisapride, cladribine, clorazepate, cromalyn, 1-deaminocysteine-8-D-arginine-vasopressin, deramciclane, detirelix, dexlansoprazole, diclofenac, didanosine, digitoxin and other digitalis glycosides, dihydrostreptomycin, dimethicone, divalproex, drospirenone, duloxetine, enzymes, erythromycin, esomeprazole, estrogens, etoposide, famotidine, fluorides, garlic oil, glucagon, granulocyte colony stimulating factor (G-CSF), heparin, hydrocortisone, human growth hormon (hGH), ibuprofen, ilaprazole, insulin, Interferon, Interleukin, Intron A, ketoprofen, lansoprazole, leuprolidacetat lipase, lipoic acid, lithium, kinin, memantine, mesalazine, methenamine, milameline, minerals, minoprazole, naproxen, natamycin, nitrofurantion, novobiocin, olsalazine, omeprazole, orothates, pancreatin, pantoprazole, parathyroidhormone, paroxetine, penicillin, perprazol, pindolol, polymyxin, potassium, pravastatin, prednisone, preglumetacin progabide, pro-somatostatin, protease, quinapril, rabeprazole, ranitidine, ranolazine, reboxetine, rutosid, somatostatin streptomycin, subtilin, sulfasalazine, sulphanilamide, tamsulosin, tenatoprazole, thrypsine, valproic acid, vasopressin, vitamins, zinc, including their salts, derivatives, polymorphs, isomorphs, acetaminophen, codeine, griseofulvin, isosorbide-5-mononitrate, lumiracoxib, metoprolol, minoxidil, nefazodone, phenytoin, remogliflozin etabonate, rivastigmine, theophylline, colchicine, darunavir, nifedipine, valacyclovir, mizoribine, ribavirin, antipyrine, glipizine, phenytoin, selegiline, theophylline, cilostazol, cimetidine, darunavir, digoxin, famotidine, fexofenadine, forskolin, indinavir, nevirapine, quinidine, ranitidine, tacrolimus, talinolol, verapamil, benazepril, cefadroxil, cephalexin, atenolol, ciprofloxacin, Fluvastatin, metformin, levodopa, gabapentin, pseudoephedrine, ropivacaine, and sotalol or mixtures or combinations thereof, preferably acetaminophen, codeine, griseofulvin, isosorbide-5-mononitrate, lumiracoxib, metoprolol, minoxidil, nefazodone, phenytoin, remogliflozin etabonate, rivastigmine, theophylline, colchicine, darunavir, nifedipine, valacyclovir, mizoribine, ribavirin, antipyrine, glipizine, phenytoin, selegiline, theophylline, cilostazol, cimetidine, darunavir, digoxin, famotidine, fexofenadine, forskolin, indinavir, nevirapine, quinidine, ranitidine, tacrolimus, talinolol, verapamil, benazepril, cefadroxil, cephalexin, atenolol, ciprofloxacin, Fluvastatin, metformin, levodopa, gabapentin, pseudoephedrine, ropivacaine, and sotalol or mixtures or combinations thereof.

It is evident to a skilled person that there is a broad overlap between the terms pharmaceutical and nutraceutical active ingredients, excipients and compositions respectively a pharmaceutical or a nutraceutical dosage form. Many substances listed as nutraceuticals may also be used as pharmaceutical active ingredients. Depending on the specific application and local authority legislation and classification, the same substance can be listed as a pharmaceutical or a nutraceutical active ingredient respectively a pharmaceutical or a nutraceutical composition or even both.

Nutraceuticals are well known to the skilled person. Nutraceuticals are often defined as extracts of foods claimed to have medical effects on human health. Thus, nutraceutical active ingredients may display pharmaceutical activities as well: Examples for nutraceutical active ingredients can be resveratrol from grape products as an antioxidant, soluble dietary fiber products, such as psyllium seed husk for reducing hypercholesterolemia, broccoli (sulphane) as a cancer preservative, and soy or clover (isoflavonoids) to improve arterial health. Thus, it is clear that many substances listed as nutraceuticals may also be used as pharmaceutical active ingredients.

Typical nutraceuticals or nutraceutical active ingredients that can be used as fill for the described polymer-coated hard shell capsules may also include probiotics and prebiotics. Probiotics are living microorganisms believed to support human or animal health when consumed. Prebiotics are nutraceuticals or nutraceutical active ingredients that induce or promote the growth or activity of beneficial microorganisms in the human or animal intestine.

Examples for nutraceuticals are resveratrol from grape products, omega-3-fatty acids or pro-anthocyanines from blueberries as antioxidants, soluble dietary fiber products, such as psyllium seed husk for reducing hypercholesterolemia, broccoli (sulphane) as a cancer preservative, and soy or clover (isoflavonoids) to improve arterial health. Other nutraceuticals examples are flavonoids, antioxidants, alpha-linoleic acid from flax seed, beta-carotene from marigold petals or antocyanins from berries. Sometimes the expression neutraceuticals or nutriceuticals are used as synonyms for nutraceuticals.

Preferred biologically active ingredients are azacytidine, decitabine, metoprolol, mesalamine and omeprazole.

### Additives

Additives according to the present invention are preferably excipients, which are well known to a skilled person and often formulated along with the biologically active ingredient contained in the coated hard shell capsule and/or with a polymer coating layer of the hard shell capsule as disclosed and claimed herein. All excipients used must be toxicologically safe and be used in pharmaceuticals or nutraceuticals without risk for patients or consumers.

The dosage form may comprise excipients, preferably pharmaceutical or nutraceutical acceptable excipients, selected from the group of antioxidants, brighteners, binding agents, flavouring agents, flow aids, fragrances, penetration-promoting agents, pore-forming agents or stabilizers or combinations thereof. The pharmaceutically or nutraceutically acceptable excipients can be comprised in the core and/or in the coating layer comprising the polymer as disclosed. A pharmaceutical or nutraceutical acceptable excipient is an excipient, which is allowed to be used for the application in the pharmaceutical or nutraceutical field.

The intermediate, enteric, or top coating layer may comprise up to 90, up to 80, up to 70, up to 50, up to 60, up to 50, up to 40, up to 30, up to 20, up to 10, up to 5 % up to 3 %, up to 1 % by weight or not any (0 %) additives at all, preferably pharmaceutically or nutraceutically acceptable excipients, based on the total weight of the at least one polymer.

### Plasticizers

The intermediate coating layer and/or the enteric coating layer can comprise one or more plasticizers. Plasticizers achieve through physical interaction with a polymer a reduction in the glass transition temperature and promote film formation, depending on the added amount. Suitable substances usually have a molecular weight of between 90 and 20,000 g/mol and comprise one or more hydrophilic groups in the molecule, e.g., hydroxyl, ester or amino groups.

Examples of suitable plasticizers are alkyl citrates, alkyl phthalates, alkyl sebacates, diethyl sebacate, dibutyl sebacate, polyethylene glycols, and polypropylene glycols. Preferred plasticizers are triethyl citrate (TEC), acetyl triethyl citrate (ATEC), diethyl sebacate, dibutyl sebacate (DBS), polyethylene glycols, and polypropylene glycols or mixtures thereof.

Addition of the plasticizers to the formulation can be carried out in a known manner, directly, in aqueous solution or after thermal pre-treatment of the mixture. It is also possible to employ mixtures of plasticizers. The enteric coating layer can comprise one or more plasticizers, preferably up to 60, up to 30, up to 25, up to 20, up to 15, up to 10, up to 5, less than 5% by weight, calculated on the at least one polymer, of a plasticizer or any (0 %) plasticizer at all can be comprised.

### Fillers

Standard fillers are usually added to the inventive formulation during processing to coating and binding agents. The quantities introduced and the use of standard fillers in pharmaceutical coatings or over layers is familiar to those skilled in the art. Examples of standard fillers are release agents, stabilizers, antioxidants, pore-forming agents, penetration-promoting agents, brighteners, fragrances or flavoring agents. They are used as processing adjuvants and are intended to ensure a reliable and reproducible preparation process as well as good long-term storage stability, or they achieve additional advantageous properties in the pharmaceutical form. They are added to the polymer formulations before processing and can influence the permeability of the coatings. This property can be used, if necessary, as an additional control parameter.

### Dosage form

The dosage form comprises the core coated with the intermediate coating layer and the enteric coating layer as disclosed and preferably can be in the form of a (coated) pellet (core) or a (coated) hard capsule (core).

The dosage form may have the form of, for instance, a tablet, a mini-tablet, a pellet, a pill, a granule, a sachet or a capsule, preferably a hard capsule. The dosage form may as well be contained, preferably in multi-units, for instance, in a tablet, in a sachet or in a capsule.

### Core

The core of the dosage form comprises a biologically active ingredient.

The core of the dosage form may comprise the biologically active ingredient distributed in a matrix structure or bound in a binder in a coating on an inner core structure or enclosed in a capsule.

The core may be prepared by methods such as granulation, extrusion, spheronization or hot melt extrusion.

The core may be a pellet, a pill, a granule, a tablet, mini-tablet or a capsule. The core may be an active ingredient-containing tablet, a pellet-containing compressed tablet, a mini-tablet or a capsule (hard or soft), which may be filled with active ingredient-containing pellets or granules, with a drug solution or dispersion, with mini-tablets or powder or combinations thereof.

The core may comprise for instance an uncoated pellet, a neutral carrier pellet, for instance a sugar sphere or non-pareilles, on top of which the biologically active ingredient is bound in a binder, such as lactose, polyvinyl pyrrolidone or a neutral cellulose-derivates such as HPC or HPMC. The binder-coating layer with the biologically active ingredient is considered herein as part of the core. The binder-coating layer of the core has, in contrast to the intermediate coating layer and the enteric coating layer, essentially no influence on the controlled release of the biologically active ingredient. The core may as well comprise an uncoated pellet consisting of a crystallized biologically active ingredient.

The core may comprise 0.1 to 100, 1 to 100, 2 to 90, 5 to 85, 10 to 70, 15 to 50 % by weight of the biologically active ingredient. The core may comprise 0 to 99.9, 0 to 99, 10 to 98, 15 to 95, 30 to 90 or 50 to 85 % by weight of the biologically active ingredient.

The core can preferably be a hard shell capsule. Hard shell capsules for pharmaceutical or nutraceutical purposes are well known to a skilled person. A hard shell capsule is a two-piece encapsulation capsule comprising of the two capsule halves, called the body and the cap. The capsule body and cap material is usually made from a hard and sometimes brittle material. The hard shell capsule comprises a body and a cap. Body and cap are usually of a one end open cylindrical form with closed rounded hemispherical ends on the opposite end. The shape and size of the cap and body are such that the body can be pushed telescopically with its open end into the open end of the cap.

The body and the cap comprise a potential overlapping, matching area (overlap area) outside the body and inside the cap which partially overlap when the capsule is closed in the pre-locked state and totally overlap in the final-locked state. When the cap is partially slid over the overlapping matching area of the body then the capsule is in the pre-locked state. When the cap is totally slid over the overlapping matching area of the body then the capsule is in the final-locked state. The maintenance of the pre-locked state or of the final-locked state is usually supported by snap-in locking mechanisms of the body and the cap such as matching encircling notches or dimples, preferably elongated dimples.

Usually, the body is longer than the cap. The outside overlapping area of the body can be covered by the cap in order to close or to lock the capsule. In the closed state the cap covers the outside overlap area of the body either in a pre-locked state or in a final-locked state. In the final-locked state the cap covers the outside overlap area of the body in total, in the pre-locked state the cap overlaps the outside overlapping area of the body only partially. The cap can be slid over the body to be fixed in usually one of two different positions in which the capsule is closed either in a pre-locked state or in a final-locked state.

Hard shell capsules are commercially available in different sizes. Hard shell capsules are usually delivered as empty containers with the body and cap already positioned in the pre-locked state and on demand as separate capsules halves, bodies and caps. The pre-locked hard shell capsules can be provided to a capsule-filling machine, which performs the opening, filling and closing of the capsule into the final-locked state. Usually, hard shell capsules are filled with dry materials, for instance with powders or granules or pellets or mini tablets or sub-micron particles, or viscous liquids comprising a biologically active ingredient.

The cap and body are provided with closure means that are advantageous for the pre-locking (temporary) and/or final locking of the capsule. Therefore, elevated points can be provided on the inner wall of the cap and somewhat larger indented points are provided on the outer wall of the body, which are arranged so that when the capsule is closed the elevations fit into the indentations. Alternatively, the elevations can be formed on the outer wall of the body and the indentations on the inner wall of the cap. Arrangements in which the elevations or indentations arranged in a ring or spiral around the wall. Instead of the point-like configuration of the elevations and indentations, these may encircle the wall of the cap or body in an annular configuration, although advantageously recesses and openings are provided which enable an exchange of gases into and out of the capsule interior. One or more elevations can be provided in an annular arrangement around the inner wall of the cap and the outer wall of the body such that, in the final-locked position of the capsule, an elevation on the cap is located adjacent to an elevation on the body. Sometimes elevations are formed on the outside of the body close to the open end and indentations are formed in the cap close to the open end such that the elevations on the body latch into the indentations in the cap in the final-locked position of the capsule. The elevations can be such that the cap can be opened in the pre-locked state at any time without damage to the capsule or, alternatively, so that once it has been closed the capsule cannot be opened again without destroying it. Capsules with one or more such latching mechanisms (latches) (for example two encircling grooves) are preferred. More preferred are capsules with at least two such latching means which secure the two capsule parts to different degrees. In a part of this kind, a first latching (dimples or encircling notches) means can be formed close to the openings in the capsule cap and the capsule body and a second latching (encircling notches) can be shifted somewhat further towards the closed end of the capsule parts. The first latching means secures the two capsule parts less strongly than the second does. This variant has the advantage that after the production of the empty capsules the capsule cap and capsule body can initially be pre-locked joined together using the first latching mechanism. In order to fill the capsule the two capsule parts are then separated again. After filling, the two capsule parts are pushed together until the second set of latches firmly secures the capsule parts in a final-locked state.

Preferably, the body and the cap of the hard shell capsule are comprising each encircling notches and/or dimples in the area, where the cap can be slid over the body. Encircling notches of the body and dimples of the cap match to each other to provide a snap-in or snap into-place mechanism. The dimples can be circular or elongated (oval) in the longitudinal direction. Encircling notches of the body and encircling notches of the cap (closely matched rings) also match to each other to provide a snap-in or snap into-place mechanism. This allows the capsule to be closed by a snap-into-place mechanism either in a pre-locked state or in a final-locked state.

Preferably, matching encircling notches of the body and elongated dimples of the cap are used to fix the body and the cap to each other in the pre-locked state. Matching encircling notches of the body and the cap are preferably used to fix or lock the body and the cap to each other in the final-locked state.

The area, where the cap can be slid over the body can be called the overlapping area of the body and the cap or briefly the overlap area. If the cap overlaps the body only partially, maybe to 20 to 90 or 60 to 85 % of the overlap area, the hard shell capsule is only partially closed (pre-locked). Preferably, in the presence of a locking mechanism, like matching encircling notches and/or dimples in body and cap, the partially closed capsule can be called pre-locked. When the capsule is polymer-coated in the pre-locked state the coating will cover the completely outer surface including that part of the overlap area of the body and cap that is not overlapped by the cap in this pre-locked state. When the capsule is polymer-coated in the pre-locked state and then closed to the final-locked state the coating of that part of the overlap area of the body and cap that was not overlapped by the cap in the pre-locked state will then become covered by the cap. The presence of that part of the coating which is then enclosed in the final-locked state between the body and the cap is sufficient for the hard shell capsule to be tightly sealed.

If the cap overlaps the body the total overlapping area of the body, the hard shell capsule is finally closed or in the final-locked state. Preferably, in the presence of a locking mechanism, like matching encircling notches and/or dimples in body and cap, the finally closed capsule can be called final-locked.

Usually, dimples are preferred for the fixing the body and the cap in the pre-locked state. As a non-binding rule the matching area of dimples is smaller than the matching area of encircling notches. Thus snapped-in dimples can be snapped-out again by applying less forces than those that would be necessary to snap-out a snapped-in fixation by matching encircling notches.

The dimples of the body and cap are located in the area, where the cap can be slid over the body match to each other in the pre-locked state by a snap in or snap into-place mechanism. There can be for example 2, 4, or preferably 6 notches or dimples located distributed circular around the cap.

Usually, the dimples of the cap are and the encircling notches of the body in the area, where the cap can be slid over the body match to each other so that they that allow the capsule to be closed by a snap-into-place mechanism in the pre-locked state. In the pre-locked state, the hard shell capsule can be re-opened manually or by a machine without damaging, because the forces needed to open are comparatively low. Thus, the "pre-locked state" is sometimes designated also as "loosely capped".

Usually, the encircling notches or matching locking rings of the body and the cap in the area, where the cap can be slid over the body match to each other so that they allow the capsule to be closed by a snap-into-place mechanism in the final-locked state. In the final-locked state, the hard shell capsule cannot or can be only hardly be re-opened manually or by a machine without damaging, because the forces needed to open are comparatively high.

Usually dimples and the encircling notches are formed in the capsule body or capsule cap. When the capsule parts provided with these elevations and indentations are fitted into one another, ideally defined uniform gaps of from 10 microns to 150 microns, more particularly 20 microns to 100 microns, are formed along the contact surface between the capsule body and the capsule cap placed thereon.

Preferably, the body of the hard shell capsule comprises a tapered rim. The tapered rim prevents the rims of the body and the cap to collide and becoming damaged when the capsule is closed manually or by a machine.

In contrast to a hard shell capsule, a soft shell capsule is a welded one piece encapsulation capsule. A soft gel capsule is often made from blow molded soft gelling substances and is usually filled with liquids comprising a biologically active ingredient by injection. The invention is not concerned with welded soft shell one piece encapsulation capsules.

### Sizes of hard shell capsules

A closed, final-locked hard shell capsule can have a total length in the range from about 5 to 40 mm. The diameter of the cap can be in the range from about 1.3 to 12 mm. The diameter of the body can be in the range from about 1.2 to 11 mm. The length of the cap can be in the range from about 4 to 20 mm and that of the body in the range from 8 to 30 mm. The fill volume can be between about from 0.004 to 2 ml. The difference between the pre-locked length and the final-locked length can be about 1 to 5 mm.

Capsules can be divided into standardized sizes for example from sizes 000 to 5. A closed capsule of size 000 has, for example, a total length of about 28 mm with an outer diameter of the cap of about 9.9 mm and an outer diameter of the body of about 9.5 mm. The length of the cap is about 14 mm, that of the body about 22 mm. The fill volume is about 1.4 ml.

A closed capsule of size 5 has, for example, a total length of about 10 mm and an outer diameter of the cap of about 4.8 mm and an outer diameter of the body of about 4.6 mm. The length of the cap is about 5.6 mm, that of the body about 9.4 mm. The fill volume is about 0.13 ml.

A closed capsule of size 9 has, for example, a total length of about 8.4 mm and an outer diameter of the cap of about 2.7 mm. The fill volume is about 0.025 ml.

A size 0 capsule may show a length of about 23 to 24 mm in the pre-locked state and of about 20.5 to 21.5 mm in the final-locked state. Thus, the difference between the pre-locked length and the final-locked length can be about 2 to 3 mm.

### Process for preparing a coated hard shell capsule

Described is a process for preparing a polymer-coated hard shell capsule, wherein the hard shell capsule comprises a body and a cap, wherein in the closed state the cap overlaps the body either in a pre-locked state or in a final-locked state, wherein the hard shell capsule is provided in the pre-locked state and coated, preferably spray-coated, with the first coating solution, suspension or dispersion according to the present invention to form an intermediate coating layer, then optionally dried and coated, preferably spray-coated, with a coating solution, suspension or dispersion according to the present invention to form an enteric coating layer, which covers the outer surface of the hard shell capsule in the pre-locked state. Furthermore, a top coating layer can subsequently, after an optional drying step be applied.

In a further process step the pre-locked hard shell capsule can be provided with a fill comprising a pharmaceutical or a nutraceutical biologically active ingredient and is closed to the final-locked state.

In such a further process step the polymer-coated hard shell capsule in the pre-locked state can be opened, filled with a fill comprising at least one biologically active ingredient, and is closed in the final-locked state. This further process step is preferably performed in that the coated hard shell capsule in the pre-locked state is provided to a capsule-filling machine, which performs the opening, filling with a fill comprising at least one biologically active ingredient and closing of the polymer-coated hard shell capsule to the final-locked state.

This further process step results in a final-locked polymer-coated hard shell capsule, which is a container for at least one biologically active ingredient. The final-locked polymer-coated hard shell capsule, which as a container for at least one biologically active ingredient is preferably a pharmaceutical or nutraceutical dosage form.

The dosage form preferably comprises a polymer-coated hard shell capsule in the final-locked state containing a fill comprising at least one biologically active ingredient, wherein the polymer-coated hard shell capsule comprises coating layers according to the invention, where the coating layers cover the outer surface area of the capsule in the pre-locked state but not the overlapping area where the cap covers the body in the pre-locked state.

A coating suspension can contain an organic solvent, for instance acetone, iso-propanol or ethanol. The concentration of dry weight material in the organic solvent can be about from 5 to 50 % by weight of polymer. A suitable spraying concentration can be about 5 to 25 % by dry weight.

A coating suspension can be the dispersion of polymers in an aqueous medium, for instance water or a mixture of 80 % by weight or more of water and 20 % or less by weight of water-soluble solvents, such as acetone or isopropanol. A suitable concentration of dry weight material in the aqueous medium can be from about 5 to 50 % by weigh. A suitable spraying concentration can be about 5 to 25 % by dry weight.

The spray coating is preferably performed by spraying the coating solution or dispersion onto the pre-locked capsules in a drum coater or in a fluidized bed coating equipment.

### Process for preparing a fill for the dosage form

Suitable processes for preparing the fill for the, preferably pharmaceutical or nutraceutical, dosage form are well known to a skilled person. A suitable process for preparing the fill for the, preferably pharmaceutical or nutraceutical, dosage form as disclosed herein can be by forming a core comprising the biologically active ingredient in the form of tablets or mini-tablets by direct compression, compression of dry, wet or sintered granules, by extrusion and subsequent rounding off, by wet or dry granulation, by direct pelleting or by binding powders onto active ingredient-free beads or neutral cores or active ingredient-containing particles or pellets and applying the intermediate and enteric coating layers in the form of aqueous dispersions or organic solutions in spray processes or by fluidized bed spray granulation.

### Capsule filling machine

The polymer-coated hard-shell capsule is provided in the pre-locked state to a capsule-filling machine, which performs the steps of separating the body and the cap, filling the body with the fill and rejoining the body and the cap in the final-locked state.

The capsule filling machine used can be a capsule filling machine, preferably a fully automated capsule filling machine, that is capable to produce filled and closed capsules at a speed with an output of 1,000 or more filled and finally closed capsules per hour. Capsule filling machines, preferably fully automated capsule filling machines, are well known in the art and commercially available from several companies.

The capsule filling machine used can be preferably operated at a speed with an output of 1,000 or more, preferably 10,000 or more, 100,000 or more, 10,000 up to 500,000, filled and finally closed capsules per hour.

### Capsule filling machine general operations

Before the capsule filling process, the capsule filling machine is provided with a sufficient number or amount of pre-coated hard-shell capsules in the pre-locked state. The capsule filling machine is also provided with sufficient amounts of fill to be filled in during operation.

The hard-shell capsules in the pre-locked state may fall by gravity into feeding tubes or chutes. The capsules can be uniformly aligned by mechanically gauging the diameter differences between the cap and the body. The hard-shell capsules are then usually fed, in proper orientation, into a two-section housing or brushing.

The diameter of the upper bushing or housing is usually larger than the diameter of the capsule body bushing; thus, the capsule cap can be retained within an upper bushing while the body is pulled into a lower bushing by vacuum. Once the capsule is opened/ the body and the cap are separated, the upper and lower housing or bushing are separated to position the capsule body for filling.

The open capsule body is then filled with the fill. Various types of filling mechanisms can be applied, with respect to the different fillings such as granules, powders, pellets or mini-tablets. Capsule filling machines in general employ a variety of mechanisms to handle the various dosage ingredients as well as various numbers of filling stations. The dosing systems are usually based on volumetric or amounts of fills governed by the capsule size and capacity of the capsule body. The empty capsule manufacturers usually provide reference tables that indicate the volume capacity of their capsule body and the maximum fill weight for different capsule sizes based on the density of the fill material. After filling, the body and the cap are rejoined by the machine in the final-locked state or position.

### Use / method of use / method steps

In a suitable process for preparing a polymer-coated hard shell capsule in the closed state the cap overlaps the body either in a pre-locked state or in a final-locked state, for preparing a polymer-coated hard shell capsule, suitable as container for biologically active ingredients, comprising the steps of
a) providing the hard shell capsule is provided in the pre-locked state and
b) spray-coating with a coating solution, suspension or dispersion comprising the components which will form the intermediate coating layer, optionally drying and afterwards spray-coating with a coating solution, suspension or dispersion comprising the components which will form the enteric coating layer which covers the outer surface of the hard shell capsule in the pre-locked state, optionally drying and optionally further applying a top coating layer.

The spray-coating can be preferably applied by using a drum coater equipment or a fluidized bed coating equipment, more preferably by using a drum coater equipment. A suitable product temperature during the spray-coating process can be in the range from about 15 to 40, preferably from about 23 to 30 °C. A suitable spray rate can be in the range from about 0.3 to 17.0, preferably 0.5 to 14 [g/min/kg]. After spray-coating a drying step is included.

The polymer-coated hard shell capsule in the pre-locked state can be opened in a step c), filled with a fill comprising a biologically active ingredient in a step d), and is then closed in a step e) to the final-locked state.

Steps c) to e) can be performed manually or preferably supported by a suitable equipment, for instance a capsule-filling machine. Preferably, the coated hard shell capsule in the pre-locked state is provided to a capsule-filling machine, which performs the opening step c), the filling with a fill comprising a biologically active ingredient in step d) and the closing of the capsule to the final-locked state in step e).

The selection of the processes in all their generic or specific features and embodiments as disclosed herein can be combined without restriction with any other generic or specific selections of materials or numerical features and embodiments as disclosed herein, such as polymers, capsule materials, capsule sizes, coating thicknesses, biologically active ingredients and any other embodiments as disclosed.

### Material of the body and the cap

The base material of the body and the cap of the hard shell capsule can be selected from hydroxypropyl methyl cellulose, starch, gelatin, pullulan and a copolymer of C1- to C4-alkylester of (meth)acrylic acid and (meth)acrylic acid. Preferred are hard shell capsules where body and cap are comprising or consisting of HPMC or gelatin, most preferred is HPMC because of its good adhesion properties for the polymer coating.

### Examples

### Compounds used in experiments

**Table 1: Biologically active ingredients and excipients used in examples**

| **Sr. No.** | **Ingredient** | **Chemical Name** | **Pharmacopoeia Grade** | **Manufacturer/ Supplier** |
|---|---|---|---|---|
| 1 | Caffeine anhydrous | Caffeine anhydrous | USP | Aarti Industries Limited |
| 2 | VIVAPUR^{®} 101 | Microcrystalline Cellulose (MCC) | USP/NF, Ph. Eur., JP | JRS PHARMA GmbH + Co. KG |
| 3 | VIVAPHARM^{®} HPMC E3 | Hypromellose (HPMC) | USP/NF, Ph. Eur., JP | JRS PHARMA GmbH + Co. KG |
| 4 | EUDRAGIT^{®} L 30 D-55 | Methacrylic Acid and Ethyl Acrylate Copolymer | USP/NF, Ph. Eur., JP | Evonik Operations GmbH |
| 5 | EUDRAGIT^{®} NM 30 D | Ethyl Acrylate and Methyl Methacrylate Copolymer | USP/NF, Ph. Eur., JP | Evonik Operations GmbH |
| 6 | IMWITOR^{®} 900 K | Glycerol monostearate 40-55%, Type II | USP/NF, Ph. Eur. | Emerson Resources INC/ Evonik Operations GmbH |
| 7 | TWEEN 80-LQ-(CQ) | Polysorbate 80 | USP/NF, Ph. Eur. | Croda Europe Limited |
| 8 | Magnesium Oxide, Light | Magnesium Oxide | USP | Dead Sea Bromine Company Ltd. |
| 9 | Glycerol anhydrous, EMPROVE^{®} | Glycerol | USP/NF, Ph. Eur., JP | Merck KGaA |
| 10 | Triethyl Citrate | Triethyl Citrate | USP/NF, Ph. Eur. | Vertellus Holdings LLC |
| 11 | Talc | Talc | USP/NF, Ph. Eur., JP | Imerys S.A. |
| 12 | Vcaps^{®} Plus capsules, size #0 | Hypromellose Capsules | USP/NF, Ph. Eur., JP | Capsugel France SAS |
| 13 | Red Iron Oxide | Iron Oxide Red | USP/NF, Ph. Eur., JP | Venator Materials PLC |
| 14 | Brilliant Blue FCF | FD&C Blue No. 1 (E133) | USP/NF, Ph. Eur., JP | Sensient Technologies Corporation |
| 15 | Titanium dioxide 3328 USP BC | Titanium dioxide | USP/NF, Ph. Eur., JP | Kronos Canada Inc. |
| 16 | FlowLac^{®} 90 | Lactose | USP/NF, Ph. Eur., JP | MEGGLE GmbH & Co. KG |
| 17 | LIPOXOL 6,000 MED | Macrogol 6000 | USP/NF, Ph. Eur., JP | Sasol Germany GmbH |

### Example 1: (Inventive) Enteric coating of pre-locked capsules in drum coater with iron oxide red in intermediate coating layer

The enteric and top coat formulations are calculated considering a surface area in pre-locked state of 545.82 mm² and a batch size of in total 1,000 capsules (500 Capsugel V-Caps Size 0 white and 500 Capsugel V-Caps Size 0 transparent capsules).

### Intermediate Coating

VIVAPHARM^{®} HPMC E3 was thoroughly dispersed in the water while gently stirring to prevent lumping. The magnesium oxide light, glycerol anhydrous and iron oxide red were homogenized in the water using a homogenizer (e. g. Ultra Turrax) for 10 minutes. The solids content should be approximately 10%. Pour the excipient suspension slowly into the VIVAPHARM^{®} HPMC E3 solution while stirring gently with a conventional stirrer. Pass the spray suspension through a 0.3 mm sieve. The spraying suspension was gently stirred during the coating process.

**Table 2:Intermediate Coating**

| **Material** | **Composition** | **Solid Composition Percentage** | **Weight per capsule** |
|---|---|---|---|
| VIVAPHARM^{®} HPMC E3 | 0.8 mg/cm² | 44.05 % | 4.5 mg |
| Magnesium Oxide Light | 100.0 % on ds* | 44.05 % | 4.5 mg |
| Glycerol anhydrous | 25.0 % on ds* | 11.0 % | 1.1 mg |
| Iron oxide red | 2.0 % on ds* | 0.9 % | 0.1 mg |
| Demineralized Water | On demand | | |
| | | | |
| Solid content | 10.0% w/w | | |
| Total solid weight gain | 1.9 mg/cm² | | |
| Total applied polymer | 0.8 mg/cm² | | |
| | | | |
| Capsule weight uncoated | | | 100.0 mg |
| Capsule weight sub coated | | | 110.2 mg |

| | | | |
|---|---|---|---|
| *Quantity based on dry polymer substance [%] | | | |

### Enteric Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 3:Enteric Coating**

| **Material** | **Composition** | **Solid Composition Percentage** | **Weight per capsule** |
|---|---|---|---|
| EUDRAGIT^{®} L 30 D-55 | 3.29 mg/cm² | 70.9 % | 18.0 mg |
| EUDRAGIT^{®} NM 30D | 0.17 mg/cm² | 3.7% | 0.9 mg |
| Triethyl citrate | 20.0 % on ds* | 14.9 % | 3.8 mg |
| IMWITOR^{®} 900 K | 10.0 % on ds* | 7.5 % | 1.9 mg |
| Polysorbate 80 | 4.0 % on ds* | 3.0 % | 0.8 mg |
| Demineralized Water | On demand | n/a | |
| | | | |
| Solid content | 10.0% w/w | | |
| Total solid weight gain | 4.6 mg/cm² | | |
| Total applied polymer | 3.5 mg/cm² | | |
| | | | |
| Capsule weight sub coated | | | 110.2 mg |
| Capsule weight functional coated | | | 135.6 mg |

| | | | |
|---|---|---|---|
| *Quantity based on dry polymer substance [%] | | | |

### Capsule Coating Process

The capsules are coated in a fully perforated side-vended pan coating system O'Hara M10.

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

Result of this example less than 10% need high forces to sperate capsule cap and body.

### Dissolution test:

Dissolution Test (according to USP 43 <711>)
Capsule manually filled. The polymer coated pre-locked capsules were manually filled with 500mg Caffeine/Lactose Mixture 3:7, closed to the final-locked state and tested in a dissolution test.

### Equipment & Set-up:

| **Parameter** | **Condition** |
|---|---|
| Apparatus | USP Apparatus I (40 Mesh Basket) |
| Dissolution Bath Temperature | 37±0.5°C |
| Basket speed | 75 rpm |
| Acid stage (Optional) | 900 mL 0.1N HCl |
| Buffer Stage | 900 mL Phosphate buffer pH 5.5 |
| Detection Wavelength | 272nm |
| Data System | OptDiss 405 UV Fiber Optic |
| Instrument ID | CCD-DI-10027 |
| Dissolution Bath | Distek 2500 |
| Diluent for preparation of Caffeine Standard solution | 100% Methanol |

### Composition and Preparation of dissolution mediums

### 0.1 N HCl (1 liter):

1. Mix 8.5 milliliters of concentrated hydrochloric acid in 1000 milliliters of de-ionized water and mix well.
2. Check the pH and adjust it to 1.2 if requires.

### Phosphate buffer pH 5.5 (10 liters):

1. Mix and dilute up to 108.87 grams of Potassium Phosphate Monohydrate in to 4 Liters of DI Water and label as 0.2 M Solution of Pot. Phosphate Monobasic Monohydrate Solution.
2. To prepare a 0.2 M of NaOH solution, take 200 ml of 1.0 N NaOH solution and dilute up to 1 Liter.
3. Take 2.5 Liter of Potassium Phosphate Solution in to 10 Lit Container. To that add 180 ml of 0.2 M NaOH solution.
4. Dilute a volume up to 10 Lit with DI water and check the pH and adjust it to the pH 5.5 with 1N NaOH or 1N HCl solution.

### Dissolution Procedure:

Acid Stage: Accurately weighed three caffeine capsules were transferred in different dissolution jars and then the dissolution test was performed as per parameters given in the method above (Acid Stage). After 2 hours 10 mL of aliquot was removed and analyzed as acid stage sample solution.

Buffer Stage: The capsules after acid stage were transferred to buffer stage medium. The dissolution test was continued as per parameters given in the method above (Buffer Stage). The aliquots of each interval ware filtered through 0.45µm nylon membrane syringe filter discarding first few mL of the filtrate and analyzed as buffer stage sample solution using following chromatographic conditions.
1. Dissolution of Caffeine Capsules (white capsules) in 900mL of 0.1N HCl for 2 Hours followed by in 900mL of pH 5.5 Phosphate Buffer

| Media | **Time (min)** | **Sample 1** | **Sample 2** | **Sample 3** | **Average** |
|---|---|---|---|---|---|
| 0.1 N HCL | 0 | 0 | 0 | 0 | 0 |
| 0.1 N HCL | 120 | 0 | 0 | 0 | 0.0 |
| pH 5.5 | 125 | 0.2 | 0.4 | 0.3 | 0.3 |
| pH 5.5 | 130 | 1.8 | 0.8 | 6.6 | 3.1 |
| pH 5.5 | 135 | 27.4 | 1.5 | 35.2 | 21.4 |
| pH 5.5 | 150 | 99.8 | 100.4 | 93.7 | 98.0 |
| pH 5.5 | 165 | 100.2 | 100.8 | 103.2 | 101.4 |
| pH 5.5 | 180 | 100.2 | 100.8 | 103.3 | 101.4 |

2. Dissolution of Caffeine Capsules (transparent capsules) in 900mL of 0.1N HCl for 2 Hours followed by in 900mL of pH 5.5 Phosphate Buffer

| Media | **Time (Min)** | **Sample 1** | **Sample 2** | **Sample 3** | **Average** |
|---|---|---|---|---|---|
| 0.1 N HCL | 0.0 | 0.0 | 0.0 | 0.0 | 0.0 |
| 0.1 N HCL | 120 | 0.0 | 0.0 | 0.0 | 0.0 |
| pH 5.5 | 125 | 1.1 | 0.4 | 0.7 | 0.7 |
| pH 5.5 | 130 | 3.2 | 2.6 | 19.7 | 8.5 |
| pH 5.5 | 135 | 6.3 | 26.5 | 47.5 | 26.8 |
| pH 5.5 | 150 | 94.1 | 99.5 | 98.7 | 97.4 |
| pH 5.5 | 165 | 101.3 | 102.7 | 102.1 | 102.0 |
| pH 5.5 | 180 | 101.3 | 102.9 | 102 | 102.1 |

### Example 2: (Inventive) Enteric coating of pre-locked capsules in drum coater with increased amount iron oxide red in intermediate coating layer

The enteric and top coat formulations are calculated considering a surface area in pre-locked state of 545.82 mm² and a batch size of in total 1,000 capsules (500 Capsugel V-Caps Size 0 white and 500 Capsugel V-Caps Size 0 transparent capsules).

### Intermediate Coating

VIVAPHARM^{®} HPMC E3 was thoroughly dispersed in the water while gently stirring to prevent lumping. The magnesium oxide light, glycerol anhydrous and iron oxide red were homogenized in the water using a homogenizer (e. g. Ultra Turrax) for 10 minutes. The solids content should be approximately 10%. Pour the excipient suspension slowly into the VIVAPHARM^{®} HPMC E3 solution while stirring gently with a conventional stirrer. Pass the spray suspension through a 0.3 mm sieve. The spraying suspension was gently stirred during the coating process.

**Table 4:Intermediate Coating**

| **Material** | **Composition** | **Solid Composition Percentage** | **Weight per capsule** |
|---|---|---|---|
| VIVAPHARM^{®} HPMC E3 | 0.8 mg/cm² | 42.0 % | 4.5 mg |
| Magnesium Oxide Light | 100.0 % on ds* | 42.0 % | 4.5 mg |
| Glycerol anhydrous | 25.0 % on ds* | 10.5 % | 1.1 mg |
| Iron oxide red | 13.0 % on ds* | 5.5 % | 0.6 mg |
| Demineralized Water | On demand | | |
| | | | |
| Solid content | 10.0% w/w | | |
| Total solid weight gain | 2.0 mg/cm² | | |
| Total applied polymer | 0.8 mg/cm² | | |
| | | | |
| Capsule weight uncoated | | | 100.0 mg |
| Capsule weight coated | | | 110.6 mg |

### Enteric Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 5:Enteric Coating**

| **Material** | **Composition** | **Solid Composition Percentage** | **Weight per capsule** |
|---|---|---|---|
| EUDRAGIT^{®} L 30 D-55 | 3.29 mg/cm² | 70.9 % | 18.0 mg |
| EUDRAGIT^{®} NM 30D | 0.17 mg/cm² | 3.7% | 0.9 mg |
| Triethyl citrate | 20.0 % on ds* | 14.9 % | 3.8 mg |
| IMWITOR^{®} 900 K | 10.0 % on ds* | 7.5 % | 1.9 mg |
| Polysorbate 80 | 4.0 % on ds* | 3.0 % | 0.8 mg |
| Demineralized Water | On demand | n/a | |
| | | | |
| Solid content | 10.0% w/w | | |
| Total solid weight gain | 4.6 mg/cm² | | |
| Total applied polymer | 3.5 mg/cm² | | |
| | | | |
| Capsule weight sub coated | | | 110.6 mg |
| Capsule weight functional coated | | | 136.0 mg |

| | | | |
|---|---|---|---|
| *Quantity based on dry polymer substance [%] | | | |

### Capsule Coating Process

The capsules are coated in a fully perforated side-vended pan coating system O'Hara M10.

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

Result of this example less than 10% need high forces to sperate capsule cap and body.

### Dissolution test:

Dissolution Test (according to USP 43 <711>)
Capsule manually filled. The polymer coated pre-locked capsules were manually filled with 500mg Caffeine/Lactose Mixture 3:7, closed to the final-locked state and tested in a dissolution test.

### Equipment & Set-up:

| **Parameter** | **Condition** |
|---|---|
| Apparatus | USP Apparatus I (40 Mesh Basket) |
| Dissolution Bath Temperature | 37±0.5°C |
| Basket speed | 75 rpm |
| Acid stage (Optional) | 900 mL 0.1N HCl |
| Buffer Stage | 900 mL Phosphate buffer pH 5.5 |
| Detection Wavelength | 272nm |
| Data System | OptDiss 405 UV Fiber Optic |
| Instrument ID | CCD-DI-10027 |
| Dissolution Bath | Distek 2500 |
| Diluent for preparation of Caffeine Standard solution | 100% Methanol |

### Composition and Preparation of dissolution mediums

### 0.1 N HCl (1 liter):

1. Mix 8.5 milliliters of concentrated hydrochloric acid in 1000 milliliters of de-ionized water and mix well.
2. Check the pH and adjust it to 1.2 if requires.

### Phosphate buffer pH 5.5 (10 liters):

1. Mix and dilute up to 108.87 grams of Potassium Phosphate Monohydrate in to 4 Liters of DI Water and label as 0.2 M Solution of Pot. Phosphate Monobasic Monohydrate Solution.
2. To prepare a 0.2 M of NaOH solution, take 200 ml of 1.0 N NaOH solution and dilute up to 1 Liter.
3. Take 2.5 Liter of Potassium Phosphate Solution in to 10 Lit Container. To that add 180 ml of 0.2 M NaOH solution.
4. Dilute a volume up to 10 Lit with DI water and check the pH and adjust it to the pH 5.5 with 1N NaOH or 1N HCl solution.

### Dissolution Procedure:

Acid Stage: Accurately weighed three caffeine capsules were transferred in different dissolution jars and then the dissolution test was performed as per parameters given in the method above (Acid Stage). After 2 hours 10 mL of aliquot was removed and analyzed as acid stage sample solution.

Buffer Stage: The capsules after acid stage were transferred to buffer stage medium. The dissolution test was continued as per parameters given in the method above (Buffer Stage). The aliquots of each interval ware filtered through 0.45µm nylon membrane syringe filter discarding first few mL of the filtrate and analyzed as buffer stage sample solution using following chromatographic conditions.
1. Dissolution of caffeine capsules (white capsules) in 900ml of 0.1n HCl for 2 hours followed by in 900ml of pH 5.5 phosphate buffer

| Media | **Time (min)** | **Sample 1** | **Sample 2** | **Sample 3** | **Average** |
|---|---|---|---|---|---|
| 0.1 N HCL | 0 | 0 | 0 | 0 | 0 |
| 0.1 N HCL | 120 | 0 | 0 | 0 | 0.0 |
| pH 5.5 | 125 | 33.4 | 58.8 | 60.3 | 50.8 |
| pH 5.5 | 130 | 62.4 | 99.9 | 74.1 | 78.8 |
| pH 5.5 | 135 | 97.5 | 105.1 | 83.4 | 95.3 |
| pH 5.5 | 150 | 106.1 | 106 | 105.9 | 106.0 |
| pH 5.5 | 165 | 106.3 | 106.1 | 105.9 | 106.1 |

### Example 3: (Inventive) Enteric coating of pre-locked capsules in drum coater with FD&C Blue No. 1 (E133) in intermediate coating layer

The enteric and top coat formulations are calculated considering a surface area in pre-locked state of 545.82 mm² and a batch size of in total 1,000 capsules (500 Capsugel V-Caps Size 0 white and 500 Capsugel V-Caps Size 0 transparent capsules).

### Intermediate Coating

VIVAPHARM^{®} HPMC E3 was thoroughly dispersed in the water while gently stirring to prevent lumping. The magnesium oxide light, glycerol anhydrous and FD&C Blue No. 1 were homogenized in the water using a homogenizer (e. g. Ultra Turrax) for 10 minutes. The solids content should be approximately 10%. Pour the excipient suspension slowly into the VIVAPHARM^{®} HPMC E3 solution while stirring gently with a conventional stirrer. Pass the spray suspension through a 0.3 mm sieve. The spraying suspension was gently stirred during the coating process.

**Table 6:Intermediate Coating**

| **Material** | **Composition** | **Solid Composition Percentage** | **Weight per capsule** |
|---|---|---|---|
| VIVAPHARM^{®} HPMC E3 | 0.8 mg/cm² | 44.05 % | 4.5 mg |
| Magnesium Oxide Light | 100.0 % on ds* | 44.05 % | 4.5 mg |
| Glycerol anhydrous | 25.0 % on ds* | 11.0 % | 1.1 mg |
| FD&C Blue No. 1 | 2.0 % on ds* | 0.9 % | 0.1 mg |
| Demineralized Water | On demand | | |
| | | | |
| Solid content | 10.0% w/w | | |
| Total solid weight gain | 1.9 mg/cm² | | |
| Total applied polymer | 0.8 mg/cm² | | |
| | | | |
| Capsule weight uncoated | | | 100.0 mg |
| Capsule weight sub coated | | | 110.2 mg |

### Enteric Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 7:Enteric Coating**

| **Material** | **Composition** | **Solid Composition Percentage** | **Weight per capsule** |
|---|---|---|---|
| EUDRAGIT^{®} L 30 D-55 | 3.29 mg/cm² | 70.9 % | 18.0 mg |
| EUDRAGIT^{®} NM 30D | 0.17 mg/cm² | 3.7% | 0.9 mg |
| Triethyl citrate | 20.0 % on ds* | 14.9 % | 3.8 mg |
| IMWITOR^{®} 900 K | 10.0 % on ds* | 7.5 % | 1.9 mg |
| Polysorbate 80 | 4.0 % on ds* | 3.0 % | 0.8 mg |
| Demineralized Water | On demand | n/a | |
| | | | |
| Solid content | 10.0% w/w | | |
| Total solid weight gain | 4.6 mg/cm² | | |
| Total applied polymer | 3.5 mg/cm² | | |
| | | | |
| Capsule weight sub coated | | | 110.2 mg |
| Capsule weight functional coated | | | 135.6 mg |

| | | | |
|---|---|---|---|
| *Quantity based on dry polymer substance [%] | | | |

### Capsule Coating Process

The capsules are coated in a fully perforated side-vended pan coating system O'Hara M10.

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

Result of this example less than 10% need high forces to sperate capsule cap and body.

### Dissolution test:

Dissolution Test (according to USP 43 <711>)
Capsule manually filled. The polymer coated pre-locked capsules were manually filled with 500mg Caffeine/Lactose Mixture 3:7, closed to the final-locked state and tested in a dissolution test.

### Equipment & Set-up:

| **Parameter** | **Condition** |
|---|---|
| Apparatus | USP Apparatus I (40 Mesh Basket) |
| Dissolution Bath Temperature | 37±0.5°C |
| Basket speed | 75 rpm |
| Acid stage (Optional) | 900 mL 0.1N HCl |
| Buffer Stage | 900 mL Phosphate buffer pH 5.5 |
| Detection Wavelength | 272nm |
| Data System | OptDiss 405 UV Fiber Optic |
| Instrument ID | CCD-DI-10027 |
| Dissolution Bath | Distek 2500 |
| Diluent for preparation of Caffeine Standard solution | 100% Methanol |

### Composition and Preparation of dissolution mediums

### 0.1 N HCl (1 liter):

1. Mix 8.5 milliliters of concentrated hydrochloric acid in 1000 milliliters of de-ionized water and mix well.
2. Check the pH and adjust it to 1.2 if requires.

### Phosphate buffer pH 5.5 (10 liters):

1. Mix and dilute up to 108.87 grams of Potassium Phosphate Monohydrate in to 4 Liters of DI Water and label as 0.2 M Solution of Pot. Phosphate Monobasic Monohydrate Solution.
2. To prepare a 0.2 M of NaOH solution, take 200 ml of 1.0 N NaOH solution and dilute up to 1 Liter.
3. Take 2.5 Liter of Potassium Phosphate Solution in to 10 Lit Container. To that add 180 ml of 0.2 M NaOH solution.
4. Dilute a volume up to 10 Lit with DI water and check the pH and adjust it to the pH 5.5 with 1N NaOH or 1N HCl solution.

### Dissolution Procedure:

Acid Stage: Accurately weighed three caffeine capsules were transferred in different dissolution jars and then the dissolution test was performed as per parameters given in the method above (Acid Stage). After 2 hours 10 mL of aliquot was removed and analyzed as acid stage sample solution.

Buffer Stage: The capsules after acid stage were transferred to buffer stage medium. The dissolution test was continued as per parameters given in the method above (Buffer Stage). The aliquots of each interval ware filtered through 0.45µm nylon membrane syringe filter discarding first few mL of the filtrate and analyzed as buffer stage sample solution using following chromatographic conditions.
1. Dissolution of Caffeine Capsules (transparent capsules) in 900mL of 0.1N HCl for 2 Hours followed by in 900mL of pH 5.5 Phosphate Buffer at 75 RPM USP I (40 mesh)

| Media | **Time (min)** | **Sample 1** | **Sample 2** | **Sample 3** | **Average** |
|---|---|---|---|---|---|
| 0.1 N HCL | 0 | 0 | 0 | 0 | 0 |
| 0.1 N HCL | 120 | 0.9 | 0.4 | 0.4 | 0.6 |
| pH 5.5 | 125 | 33.4 | 58.8 | 60.3 | 50.8 |
| pH 5.5 | 130 | 62.4 | 99.9 | 74.1 | 78.8 |
| pH 5.5 | 135 | 97.5 | 105.1 | 83.4 | 95.3 |
| pH 5.5 | 150 | 106.1 | 106 | 105.9 | 106.0 |
| pH 5.5 | 165 | 106.3 | 106.1 | 105.9 | 106.1 |

### Example 4: (Inventive) Enteric coating of pre-locked capsules in drum coater with increased amount titanium dioxide in intermediate coating layer

The enteric and top coat formulations are calculated considering a surface area in pre-locked state of 545.82 mm² and a batch size of in total 1,000 capsules (500 Capsugel V-Caps Size 0 white and 500 Capsugel V-Caps Size 0 transparent capsules).

### Intermediate Coating

VIVAPHARM^{®} HPMC E3 was thoroughly dispersed in the water while gently stirring to prevent lumping. The magnesium oxide light, glycerol anhydrous and titanium dioxide were homogenized in the water using a homogenizer (e. g. Ultra Turrax) for 10 minutes. The solids content should be approximately 10%. Pour the excipient suspension slowly into the VIVAPHARM^{®} HPMC E3 solution while stirring gently with a conventional stirrer. Pass the spray suspension through a 0.3 mm sieve. The spraying suspension was gently stirred during the coating process.

**Table 8:Intermediate Coating**

| **Material** | **Composition** | **Solid Composition Percentage** | **Weight per capsule** |
|---|---|---|---|
| VIVAPHARM^{®} HPMC E3 | 0.8 mg/cm² | 42.0 % | 4.5 mg |
| Magnesium Oxide Light | 100.0 % on ds* | 42.0 % | 4.5 mg |
| Glycerol anhydrous | 25.0 % on ds* | 10.5 % | 1.1 mg |
| Titanium dioxide | 13.0 % on ds* | 5.5 % | 0.6 mg |
| Demineralized Water | On demand | | |
| | | | |
| Solid content | 10.0% w/w | | |
| Total solid weight gain | 2.0 mg/cm² | | |
| Total applied polymer | 0.8 mg/cm² | | |
| | | | |
| Capsule weight uncoated | | | 100.0 mg |
| Capsule weight coated | | | 110.6 mg |

### Enteric Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 9:Enteric Coating**

| **Material** | **Composition** | **Solid Composition Percentage** | **Weight per capsule** |
|---|---|---|---|
| EUDRAGIT^{®} L 30 D-55 | 3.29 mg/cm² | 70.9 % | 18.0 mg |
| EUDRAGIT^{®} NM 30D | 0.17 mg/cm² | 3.7% | 0.9 mg |
| Triethyl citrate | 20.0 % on ds* | 14.9 % | 3.8 mg |
| IMWITOR^{®} 900 K | 10.0 % on ds* | 7.5 % | 1.9 mg |
| Polysorbate 80 | 4.0 % on ds* | 3.0 % | 0.8 mg |
| Demineralized Water | On demand | n/a | |
| | | | |
| Solid content | 10.0% w/w | | |
| Total solid weight gain | 4.6 mg/cm² | | |
| Total applied polymer | 3.5 mg/cm² | | |
| | | | |
| Capsule weight sub coated | | | 110.6 mg |
| Capsule weight functional coated | | | 136.0 mg |

| | | | |
|---|---|---|---|
| *Quantity based on dry polymer substance [%] | | | |

### Capsule Coating Process

The capsules are coated in a fully perforated side-vended pan coating system O'Hara M10.

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

Result of this example less than 10% need high forces to sperate capsule cap and body.

### Dissolution test:

Dissolution Test (according to USP 43 <711>)
Capsule manually filled. The polymer coated pre-locked capsules were manually filled with 500mg Caffeine/Lactose Mixture 3:7, closed to the final-locked state and tested in a dissolution test.

### Equipment & Set-up:

| **Parameter** | **Condition** |
|---|---|
| Apparatus | USP Apparatus I (40 Mesh Basket) |
| Dissolution Bath Temperature | 37±0.5°C |
| Basket speed | 75 rpm |
| Acid stage (Optional) | 900 mL 0.1N HCl |
| Buffer Stage | 900 mL Phosphate buffer pH 5.5 |
| Detection Wavelength | 272nm |
| Data System | OptDiss 405 UV Fiber Optic |
| Instrument ID | CCD-DI-10027 |
| Dissolution Bath | Distek 2500 |
| Diluent for preparation of Caffeine Standard solution | 100% Methanol |

### Composition and Preparation of dissolution mediums

### 0.1 N HCl (1 liter):

1. Mix 8.5 milliliters of concentrated hydrochloric acid in 1000 milliliters of de-ionized water and mix well.
2. Check the pH and adjust it to 1.2 if requires.

### Phosphate buffer pH 5.5 (10 liters):

1. Mix and dilute up to 108.87 grams of Potassium Phosphate Monohydrate in to 4 Liters of DI Water and label as 0.2 M Solution of Pot. Phosphate Monobasic Monohydrate Solution.
2. To prepare a 0.2 M of NaOH solution, take 200 ml of 1.0 N NaOH solution and dilute up to 1 Liter.
3. Take 2.5 Liter of Potassium Phosphate Solution in to 10 Lit Container. To that add 180 ml of 0.2 M NaOH solution.
4. Dilute a volume up to 10 Lit with DI water and check the pH and adjust it to the pH 5.5 with 1N NaOH or 1N HCl solution.

### Dissolution Procedure:

Acid Stage: Accurately weighed three caffeine capsules were transferred in different dissolution jars and then the dissolution test was performed as per parameters given in the method above (Acid Stage). After 2 hours 10 mL of aliquot was removed and analyzed as acid stage sample solution.

Buffer Stage: The capsules after acid stage were transferred to buffer stage medium. The dissolution test was continued as per parameters given in the method above (Buffer Stage). The aliquots of each interval ware filtered through 0.45µm nylon membrane syringe filter discarding first few mL of the filtrate and analyzed as buffer stage sample solution using following chromatographic conditions.
1. Dissolution of Caffeine Capsules (white capsules) in 900mL of 0.1N HCl for 2 Hours followed by in 900mL of pH 5.5 Phosphate Buffer

| Media | **Time (min)** | **Sample 1** | **Sample 2** | **Sample 3** | **Average** |
|---|---|---|---|---|---|
| 0.1 N HCL | 0 | 0 | 0 | 0 | 0 |
| 0.1 N HCL | 120 | 0.2 | 0.3 | 1.6 | 0.7 |
| pH 5.5 | 125 | 19.9 | 13.2 | 47.5 | 26.9 |
| pH 5.5 | 130 | 96.3 | 30.9 | 80.1 | 69.1 |
| pH 5.5 | 135 | 105.9 | 91 | 94.8 | 97.2 |
| pH 5.5 | 150 | 106.9 | 110.4 | 106.2 | 107.8 |
| pH 5.5 | 165 | 107.2 | 110.5 | 106.3 | 108 |

### Example 5: (Inventive) Enteric coating of pre-locked capsules in drum coater with increased amount lactose in intermediate coating layer

The enteric and top coat formulations are calculated considering a surface area in pre-locked state of 545.82 mm² and a batch size of in total 1,000 capsules (500 Capsugel V-Caps Size 0 white and 500 Capsugel V-Caps Size 0 transparent capsules).

### Intermediate Coating

VIVAPHARM^{®} HPMC E3 was thoroughly dispersed in the water while gently stirring to prevent lumping. The magnesium oxide light, glycerol anhydrous and lactose were homogenized in the water using a homogenizer (e. g. Ultra Turrax) for 10 minutes. The solids content should be approximately 10%. Pour the excipient suspension slowly into the VIVAPHARM^{®} HPMC E3 solution while stirring gently with a conventional stirrer. Pass the spray suspension through a 0.3 mm sieve. The spraying suspension was gently stirred during the coating process.

**Table 10:Intermediate Coating**

| **Material** | **Composition** | **Solid Composition Percentage** | **Weight per capsule** |
|---|---|---|---|
| VIVAPHARM^{®} HPMC E3 | 0.8 mg/cm² | 42.0 % | 4.5 mg |
| Magnesium Oxide Light | 100.0 % on ds* | 42.0 % | 4.5 mg |
| Glycerol anhydrous | 25.0 % on ds* | 10.5 % | 1.1 mg |
| Lactose | 13.0 % on ds* | 5.5 % | 0.6 mg |
| Demineralized Water | On demand | | |
| | | | |
| Solid content | 10.0% w/w | | |
| Total solid weight gain | 2.0 mg/cm² | | |
| Total applied polymer | 0.8 mg/cm² | | |
| | | | |
| Capsule weight uncoated | | | 100.0 mg |
| Capsule weight coated | | | 110.6 mg |

### Enteric Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 11:Enteric Coating**

| **Material** | **Composition** | **Solid Composition Percentage** | **Weight per capsule** |
|---|---|---|---|
| EUDRAGIT^{®} L 30 D-55 | 3.29 mg/cm² | 70.9 % | 18.0 mg |
| EUDRAGIT^{®} NM 30D | 0.17 mg/cm² | 3.7% | 0.9 mg |
| Triethyl citrate | 20.0 % on ds* | 14.9 % | 3.8 mg |
| IMWITOR^{®} 900 K | 10.0 % on ds* | 7.5 % | 1.9 mg |
| Polysorbate 80 | 4.0 % on ds* | 3.0 % | 0.8 mg |
| Demineralized Water | On demand | n/a | |
| | | | |
| Solid content | 10.0% w/w | | |
| Total solid weight gain | 4.6 mg/cm² | | |
| Total applied polymer | 3.5 mg/cm² | | |
| | | | |
| Capsule weight sub coated | | | 110.6 mg |
| Capsule weight functional coated | | | 136.0 mg |

| | | | |
|---|---|---|---|
| *Quantity based on dry polymer substance [%] | | | |

### Capsule Coating Process

The capsules are coated in a fully perforated side-vended pan coating system O'Hara M10.

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

Result of this example less than 10% need high forces to sperate capsule cap and body.

### Dissolution test:

Dissolution Test (according to USP 43 <711>)
Capsule manually filled. The polymer coated pre-locked capsules were manually filled with 500mg Caffeine/Lactose Mixture 3:7, closed to the final-locked state and tested in a dissolution test.

### Equipment & Set-up:

| **Parameter** | **Condition** |
|---|---|
| Apparatus | USP Apparatus I (40 Mesh Basket) |
| Dissolution Bath Temperature | 37±0.5°C |
| Basket speed | 75 rpm |
| Acid stage (Optional) | 900 mL 0.1N HCl |
| Buffer Stage | 900 mL Phosphate buffer pH 5.5 |
| Detection Wavelength | 272nm |
| Data System | OptDiss 405 UV Fiber Optic |
| Instrument ID | CCD-DI-10027 |
| Dissolution Bath | Distek 2500 |
| Diluent for preparation of Caffeine Standard solution | 100% Methanol |

### Composition and Preparation of dissolution mediums

### 0.1 N HCl (1 liter):

1. Mix 8.5 milliliters of concentrated hydrochloric acid in 1000 milliliters of de-ionized water and mix well.
2. Check the pH and adjust it to 1.2 if requires.

### Phosphate buffer pH 5.5 (10 liters):

1. Mix and dilute up to 108.87 grams of Potassium Phosphate Monohydrate in to 4 Liters of DI Water and label as 0.2 M Solution of Pot. Phosphate Monobasic Monohydrate Solution.
2. To prepare a 0.2 M of NaOH solution, take 200 ml of 1.0 N NaOH solution and dilute up to 1 Liter.
3. Take 2.5 Liter of Potassium Phosphate Solution in to 10 Lit Container. To that add 180 ml of 0.2 M NaOH solution.
4. Dilute a volume up to 10 Lit with DI water and check the pH and adjust it to the pH 5.5 with 1N NaOH or 1N HCl solution.

### Dissolution Procedure:

Acid Stage: Accurately weighed three caffeine capsules were transferred in different dissolution jars and then the dissolution test was performed as per parameters given in the method above (Acid Stage). After 2 hours 10 mL of aliquot was removed and analyzed as acid stage sample solution.

Buffer Stage: The capsules after acid stage were transferred to buffer stage medium. The dissolution test was continued as per parameters given in the method above (Buffer Stage). The aliquots of each interval ware filtered through 0.45µm nylon membrane syringe filter discarding first few mL of the filtrate and analyzed as buffer stage sample solution using following chromatographic conditions.
1. Dissolution of caffeine capsules (white capsules) in 900ml of 0.1n HCl for 2 hours followed by in 900ml of pH 5.5 phosphate buffer

| **Media** | **Time (min)** | **Sample 1** | **Sample 2** | **Sample 3** | **Average** |
|---|---|---|---|---|---|
| 0.1 N HCL | 0.0 | 0.0 | 0.0 | 0.0 | 0.0 |
| 0.1 N HCL | 120 | 0.0 | 0.0 | 0.0 | 0.0 |
| pH 5.5 | 125 | 50.6 | 6.8 | 20.8 | 26.1 |
| pH 5.5 | 130 | 77.0 | 19.3 | 38.1 | 44.8 |
| pH 5.5 | 135 | 85.7 | 50.4 | 63.0 | 66.4 |
| pH 5.5 | 150 | 95.0 | 95.5 | 95.8 | 95.4 |
| pH 5.5 | 165 | 95.1 | 98.5 | 96.8 | 96.8 |

### Example 6: (Comparative) Enteric coating of pre-locked capsules in drum coater without release acceleration agent in intermediate coating layer

The enteric and top coat formulations are calculated considering a surface area in pre-locked state of 545.82 mm² and a batch size of in total 1,000 capsules (500 Capsugel V-Caps Size 0 white and 500 Capsugel V-Caps Size 0 transparent capsules).

### Intermediate Coating

VIVAPHARM^{®} HPMC E3 was thoroughly dispersed in the water while gently stirring to prevent lumping. The magnesium oxide light and glycerol anhydrous were homogenized in the water using a homogenizer (e. g. Ultra Turrax) for 10 minutes. The solids content should be approximately 10%. Pour the excipient suspension slowly into the VIVAPHARM^{®} HPMC E3 solution while stirring gently with a conventional stirrer. Pass the spray suspension through a 0.3 mm sieve. The spraying suspension was gently stirred during the coating process.

**Table 12:Intermediate Coating**

| **Ingredients** | **Composition** | **Solid Composition Percentage** |
|---|---|---|
| VIVAPHARM^{®} HPMC E3 | 0.89 mg/cm² | 44.44 % |
| Glycerol anhydrous | 25 % on ds* | 11.11 % |
| Magnesium Oxide Light | 100 % on ds* | 44.44 % |
| Demineralized Water | On demand | |
| | | |
| Solid content | 10.0% w/w | |
| Polymer weight gain | | 0.89 mg/cm² |
| Total weight gain | | 2.0 mg/cm² |

### Enteric Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 13:Enteric Coating**

| **Material** | **Composition** | **Solid Composition Percentage** |
|---|---|---|
| EUDRAGIT^{®} L 30 D-55 | 3.12 mg/cm² | 66.67 % |
| Triethyl citrate | 10.0 % on ds* | 6.67 % |
| Talc | 50.0 % on ds* | 33.33 % |
| Demineralized Water | On demand | n/a |
| | | |
| Solid content | 20.0% w/w | |
| Total solid weight gain | 5.0 mg/cm² | |
| Total applied polymer | 3.12 mg/cm² | |

| | | |
|---|---|---|
| *Quantity based on dry polymer substance [%] | | |

### Capsule Coating Process for intermediate and enteric coating:

| **Equipment** | | Neocota |
|---|---|---|
| **Equipment setup** | | |
| Silicone tube inner diameter | mm | 3.0 |
| Pan size | inch | 14 |
| Number of baffles | No.s | 6 |

| **Process parameter** | | |
|---|---|---|
| Pan RPM | RPM | 2 - 8 |
| Inlet temperature | °C | 45 - 50 |
| Product temperature | °C | 28 - 32 |
| Atomization pressure | bar | 1.0 - 1.5 |
| Spray rate | g/min | 3 - 5 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

Result of this example less than 10% need high forces to sperate capsule cap and body.

### Dissolution test:

### A) Dissolution Conditions

1) Dissolution Parameters

| | |
|---|---|
| Apparatus | : USP Type I |
| Dissolution Medium | : 2 hours acid stage medium followed by full change to 2 hours buffer stage medium |
| Sampling points | : Acid stage medium; 60 min, 120 min |
| | : Buffer stage medium; 10min, 20min, 30 min, 45min, 60 min, 120 min |
| Volume of Medium | : 750mL for acid stage, 1000 mL for buffer stage |
| Speed | : 100 rpm |
| Temperature | : 37°C ± 0.5°C |
| Withdrawal Volume | : 10ml |

2) Dissolution mediums
I. Acid stage medium- 0.1 N HCl; buffer stage medium - pH 5.0 buffer, or
II. Acid stage medium- 0.1 N HCl; buffer stage medium - pH 4.5 buffer, or
III. Acid stage medium- 0.1 N HCl; buffer stage medium - pH 3.0 buffer

3) Composition of buffer stage mediums
I. Buffer stage medium - pH 5.0 buffer
   1 g of Potassium dihydrogen phosphate (KH₂PO₄), 2 g of Di-potassium hydrogen phosphate anhydrous (K₂HPO₄) and 8.5 g of sodium chloride (NaCl) was weighed and transferred to 1-liter beaker. To this, 500 mL water was added, salts were dissolved, and volume was made up to 1000 mL with water. The pH was adjusted to 5.0 + 0.05 using ortho-phosphoric acid.
II. Buffer stage medium - pH 4.5 buffer
   1 g of Potassium dihydrogen phosphate (KH₂PO₄), 2 g of Di-potassium hydrogen phosphate anhydrous (K₂HPO₄) and 8.5 g of sodium chloride (NaCl) was weighed and transferred to 1-liter beaker. To this, 500 mL water was added, salts were dissolved, and volume was made up to 1000 mL with water. The pH was adjusted to 4.5 + 0.05 using ortho-phosphoric acid.
III. Buffer stage medium - pH 3.0 buffer
   1 g of Potassium dihydrogen phosphate (KH₂PO₄), 2 g of Di-potassium hydrogen phosphate anhydrous (K₂HPO₄) and 8.5 g of sodium chloride (NaCl) was weighed and transferred to 1-liter beaker. To this, 500 mL water was added, salts were dissolved, and volume was made up to 1000 mL with water. The pH was adjusted to 3.0 + 0.05 using ortho-phosphoric acid.

4) Dissolution Procedure:
Acid Stage: Accurately weighed six caffeine capsules were transferred in different dissolution jars and then the dissolution test was performed as per parameters given in the method above (Acid Stage). After 1 & 2 hours 10 mL of aliquot was removed and analyzed as acid stage sample solution.
Buffer Stage: The capsules after acid stage were transferred to buffer stage medium. The dissolution test was continued as per parameters given in the method above (Buffer Stage). The aliquots of each interval ware filtered through 0.45µm nylon membrane syringe filter discarding first few mL of the filtrate and analyzed as buffer stage sample solution using following chromatographic conditions.

### B) Chromatographic Conditions

| | |
|---|---|
| Column : | Agilent Zorbax Eclipse XDB C 18 column, 150 x 4.6 mm, 5µm or equivalent |
| Mobile Phase: | Water/ACN (80:20) |
| Wavelength: | 273 nm |
| Column Temp: | 25°C |
| Injection Volume: | 10 µL |
| Flow rate: | 1.5 mL/minute |

| **Dissolution testing in acid stage media** | |
|---|---|
| pH 1.2 (120 min) | Pass |

| **Dissolution testing (% drug release) in buffer stage media** | |
|---|---|
| pH 5.0 (45 min) | 30.0 |
| pH 5.0 (60 min) | 46.5 |
| pH 4.5 (45min) | No drug release detected |
| pH 4.5 (60min) | |
| pH 3.0 (45 min) | |
| pH 3.0 (60 min) | |

### Example 7: (inventive) Enteric coating of API minitablets in fluidized bed coater with increased amount lactose in intermediate coating layer

The enteric and top coat formulations are calculated considering a surface area of 22.14 mm² and a batch size of in total 750g API mini-tablets (diameter 2.5 mm concave).

### Intermediate Coating

VIVAPHARM^{®} HPMC E3 was thoroughly dispersed in the water while gently stirring to prevent lumping. The magnesium oxide light, glycerol anhydrous and lactose were homogenized in the water using a homogenizer (e. g. Ultra Turrax) for 10 minutes. The solids content should be approximately 15%. Pour the excipient suspension slowly into the VIVAPHARM^{®} HPMC E3 solution while stirring gently with a conventional stirrer. Pass the spray suspension through a 0.3 mm sieve. The spraying suspension was gently stirred during the coating process.

**Table 14:Intermediate Coating**

| **Material** | **Composition** | **Solid Composition Percentage** | **Weight per capsule** |
|---|---|---|---|
| VIVAPHARM^{®} HPMC E3 | 4.4 mg/cm² | 44.77 % | 1.0 mg |
| Magnesium Oxide Light | 100.0 % on ds* | 44.77 % | 1.0 mg |
| LIPOXOL 6,000 MED | 10.0 % on ds* | 4.48 % | 0.1 mg |
| Lactose | 13.4 % on ds* | 5.98 % | 0.1 mg |
| Demineralized Water | On demand | | |
| | | | |
| Solid content | 15.0% w/w | | |
| Total solid weight gain | 9.9 mg/cm² | | |
| Total applied polymer | 4.4 mg/cm² | | |
| | | | |
| Capsule weight uncoated | | | 10.0 mg |
| Capsule weight coated | | | 12.2 mg |

### Enteric Coating

The triethyl citrate and talc were homogenized in the water using a homogenizer (e. g. Ultra Turrax) for 10 minutes. Then the excipient suspension was poured slowly into the EUDRAGIT^{®} L 30 D-55 dispersion while stirring gently with a conventional stirrer and stirred for further 15 minutes. The final coating suspension was sieved throughout a 400µm sieve and stirred during the coating process.

**Table 15:Enteric Coating**

| **Material** | **Composition** | **Solid Composition Percentage** | **Weight per capsule** |
|---|---|---|---|
| EUDRAGIT^{®} L 30 D-55 | 3.6 mg/cm² | 62.50 % | 1.1 mg |
| Triethyl citrate | 10.0 % on ds* | 31.25 % | 0.6 mg |
| Talc | 50.0 % on ds* | 6.25 % | 0.1 mg |
| Demineralized Water | On demand | n/a | |
| | | | |
| Solid content | 20.0% w/w | | |
| Total solid weight gain | 5.7 mg/cm² | | |
| Total applied polymer | 3.6 mg/cm² | | |
| | | | |
| Capsule weight sub coated | | | 12.2 mg |
| Capsule weight functional coated | | | 14.0 mg |

| | | | |
|---|---|---|---|
| *Quantity based on dry polymer substance [%] | | | |

### Mini-tablet Coating Process

### Process parameters for enteric coating of 750g of minitablets using GPCG-2 system

| **EQUIPMENT SETUP:** | | | |
|---|---|---|---|
| Air distribution Plate | C | | |
| Bottom Sieve | 250 # | | |
| Finger bag | Bonnet | | |

| **Variables** | **Unit** | **Intermediate coating** | **Enteric coating** |
|---|---|---|---|
| Silicon tube ID/OD | mm | 3/5 | 3/5 |
| Filter shaking mode | - | Asynchronous | Asynchronous |
| Filter shaking time | Sec | 10 | 10 |
| Filter shaking pause | Sec | 100 | 100 |
| Area temperature | °C | NMT 25 | NMT 25 |
| Area Relative Humidity | % | NMT 60 | NMT 60 |
| Spray gun type | | Schlick | Schlick |
| Spray gun number | | 1 | 1 |
| Nozzle bore | mm | 1 | 1 |
| Inlet temperature | °C | 35-45 | 30-35 |
| Product temperature | °C | 30-38 | 25-35 |
| Air flow | CFM | 160-170 | 160-170 |
| Column height | | 15- 20 | 15-25 |
| Atomization pressure | Bar | 1.2 - 1.4 | 1.0-2.0 |
| Spray rate | g/min | 4.0 - 6.5 | 1.2-5.0 |

### Dissolution Test (according to USP 43 <711>)

### Dissolution system

| **Parameter** | **Condition** |
|---|---|
| Apparatus | USP Apparatus I (40 Mesh Basket) |
| Dissolution Bath Temperature | 37±0.5°C |
| Paddle speed | 75 rpm |
| 0.1N HCl volume | 900 mL |
| pH 3.0 HCl Volume | 900 mL |
| Dissolution Sampling Points in 0.1N HCl | 60 and 120 minutes |
| Dissolution Sampling Points in pH 3.0 HCl | 165 and 180 minutes |
| Infinity at 300 RPM | 240 minutes |
| Sampling volume | 1.5 mL |

### HPLC system parameters

| **Parameter** | **Condition** |
|---|---|
| Data System | Empower 3 Software |
| Sample Temperature | 5°C |
| Column Temperature | 23°C |
| Run Time | 12 minutes |
| HPLC Column | WATERS X-Select CSH Phenyl-Hexyl Column 4.6 x 75mm, 3.5µ With Guard Column |
| Wavelength | 200 nm |
| Injection volume | 10 µL for 20mg strength |
| Flow rate | 0.7 mL/min |
| Needle wash | ACN: De-ionized water (50:50) |

### Dissolution mediums

### 0.1N Hydrochloric acid (pH 1.2)

1. Mix 8.5 milliliters of concentrated hydrochloric acid in 1000 milliliters of de-ionized water and mix well.

### 0.001N Hydrochloric acid solution (pH 3.0)

1. Mix 1.0 milliliters of concentrated hydrochloric acid in 11.764 liters of de-ionized water and mix well.
2. If required, adjust it to pH 3.0 with 2. If required, adjust it to pH 3.0 with 0.1N HCl or 0.1N NaOH solution.

### Results

| **Dissolution testing in acid stage media** | |
|---|---|
| pH 1.2 (120 min) | Pass - less than 10% |

| **Dissolution testing (% drug release) in buffer stage media** | |
|---|---|
| pH 3.0 (30 min) | Pass - more than 80% |

### Example 8: (Comparative) Enteric coating of API minitablets in fluidized bed coater without release acceleration agent in intermediate coating layer

The enteric and top coat formulations are calculated considering a surface area of 22.14 mm² and a batch size of in total 750g API mini-tablets (diameter 2.5 mm concave).

### Intermediate Coating

VIVAPHARM^{®} HPMC E3 was thoroughly dispersed in the water while gently stirring to prevent lumping. The glycerol anhydrous and lactose were homogenized in the water using a homogenizer (e. g. Ultra Turrax) for 10 minutes. The solids content should be approximately 15%. Pour the excipient suspension slowly into the VIVAPHARM^{®} HPMC E3 solution while stirring gently with a conventional stirrer. Pass the spray suspension through a 0.3 mm sieve. The spraying suspension was gently stirred during the coating process.

**Table 16:Intermediate Coating**

| **Material** | **Composition** | **Solid Composition Percentage** | **Weight per capsule** |
|---|---|---|---|
| VIVAPHARM^{®} HPMC E3 | 4.4 mg/cm² | 47.62 % | 1.0 mg |
| Magnesium Oxide Light | 100.0 % on ds* | 47.62 % | 1.0 mg |
| LIPOXOL 6,000 MED | 10.0 % on ds* | 4.76 % | 0.1 mg |
| Demineralized Water | On demand | | |
| | | | |
| Solid content | 15.0% w/w | | |
| Total solid weight gain | 9.3 mg/cm² | | |
| Total applied polymer | 4.4 mg/cm² | | |
| | | | |
| Capsule weight uncoated | | | 10.0 mg |
| Capsule weight coated | | | 12.1 mg |

### Enteric Coating

The triethyl citrate and talc were homogenized in the water using a homogenizer (e. g. Ultra Turrax) for 10 minutes. Then the excipient suspension was poured slowly into the EUDRAGIT^{®} L 30 D-55 dispersion while stirring gently with a conventional stirrer and stirred for further 15 minutes. The final coating suspension was sieved throughout a 400µm sieve and stirred during the coating process.

**Table 17:Enteric Coating**

| **Material** | **Composition** | **Solid Composition Percentage** | **Weight per capsule** |
|---|---|---|---|
| EUDRAGIT^{®} L 30 D-55 | 4.1 mg/cm² | 62.50 % | 1.1 mg |
| Triethyl citrate | 10.0 % on ds* | 31.25 % | 0.6 mg |
| Talc | 50.0 % on ds* | 6.25 % | 0.1 mg |
| Demineralized Water | On demand | n/a | |
| | | | |
| Solid content | 20.0% w/w | | |
| Total solid weight gain | 6.6 mg/cm² | | |
| Total applied polymer | 4.1 mg/cm² | | |
| | | | |
| Capsule weight sub coated | | | 12.1 mg |
| Capsule weight functional coated | | | 13.7 mg |

| | | | |
|---|---|---|---|
| *Quantity based on dry polymer substance [%] | | | |

### Mini-tablet Coating Process

### Process parameters for enteric coating of 750g of minitablets using GPCG-2 system

| **EQUIPMENT SETUP:** | | | |
|---|---|---|---|
| Air distribution Plate | C | | |
| Bottom Sieve | 250 # | | |
| Finger bag | Bonnet | | |

| **Variables** | **Unit** | **Intermediate coating** | **Enteric coating** |
|---|---|---|---|
| Silicon tube ID/OD | mm | 3/5 | 3/5 |
| Filter shaking mode | - | Asynchronous | Asynchronous |
| Filter shaking time | Sec | 10 | 10 |
| Filter shaking pause | Sec | 100 | 100 |
| Area temperature | °C | NMT 25 | NMT 25 |
| Area Relative Humidity | % | NMT 60 | NMT 60 |
| Spray gun type | | Schlick | Schlick |
| Spray gun number | | 1 | 1 |
| Nozzle bore | mm | 1 | 1 |
| Inlet temperature | °C | 35-45 | 30-35 |
| Product temperature | °C | 30-38 | 25-35 |
| Air flow | CFM | 160-170 | 160-170 |
| Column height | | 15- 20 | 15-25 |
| Atomization pressure | Bar | 1.2 - 1.4 | 1.0-2.0 |
| Spray rate | g/min | 4.0 - 6.5 | 1.2-5.0 |

### Dissolution Test (according to USP 43 <711>)

### Dissolution system

| **Parameter** | **Condition** |
|---|---|
| Apparatus | USP Apparatus I (40 Mesh Basket) |
| Dissolution Bath Temperature | 37±0.5°C |
| Paddle speed | 75 rpm |
| 0.1N HCl | 900 mL |
| pH 3.0 HCl | 900 mL |
| Dissolution Sampling Points in 0.1N HCl | 60 and 120 minutes |
| Dissolution Sampling Points in pH 3.0 HCl | 165 and 180 minutes |
| Infinity at 300 RPM | 240 minutes |
| Sampling volume | 1.5 mL |

### HPLC system parameters

| **Parameter** | **Condition** |
|---|---|
| Data System | Empower 3 Software |
| Sample Temperature | 5°C |
| Column Temperature | 23°C |
| Run Time | 12 minutes |
| HPLC Column | WATERS X-Select CSH Phenyl-Hexyl Column 4.6 x 75mm, 3.5µ With Guard Column |
| Wavelength | 200 nm |
| Injection volume | 10 µL for 20mg strength |
| Flow rate | 0.7 mL/min |
| Needle wash | ACN: De-ionized water (50:50) |

### Dissolution mediums

### 0.1N Hydrochloric acid (pH 1.2)

1. Mix 8.5 milliliters of concentrated hydrochloric acid in 1000 milliliters of de-ionized water and mix well.

### 0.001N Hydrochloric acid solution (pH 3.0)

1. Mix 1.0 milliliters of concentrated hydrochloric acid in 11.764 liters of de-ionized water and mix well.
2. If required, adjust it to pH 3.0 with 2. If required, adjust it to pH 3.0 with 0.1N HCl or 0.1N NaOH solution.

### Results

| **Dissolution testing in acid stage media** | |
|---|---|
| pH 1.2 (120 min) | Pass - less than 10% |

| **Dissolution testing (% drug release) in buffer stage media** | |
|---|---|
| pH 3.0 (45 min) | Fail - less than 80% |
| pH 3.0 (60 min) | Fail - less than 80% |

### Example 9: (inventive) Manufacturing of Caffeine minitablets; followed by Seal, Intermediate and Enteric coating of Caffeine minitablets (enteric with 6.25 % weight gain of EUDRAGIT L30D-55)

Tablet Compression: Formulation blend of Caffeine, MCC, Lactose, Aerosil 200 and Croscarmellose Sodium is prepared using Turbula mixer. Followed by addition of Magnesium Stearate as lubricant with total blending time of 15 minutes. This blend is compressed by 2.5mm multi tip tooling using Korsch XL compression machines. Compression force of about 17KN is utilized to produced 2.5mm round minitablets weighing 10.0mg, thickness of 1.8mm and Hardness of 3.02KP.

### Formulation Composition:

| Ingredient | Function | Amount (%) | Amount (g) |
|---|---|---|---|
| Caffeine | API | 20 | 200 |
| Lactose monohydrate | Diluent/Filler | 48.5 | 485 |
| Croscarmellose Sodium | Super-disintegrant | 5 | 50 |
| Microcrystalline Cellulose PH-200 | Diluent/Filler | 25 | 250 |
| Aerosil | Glidant | 1 | 10 |
| Magnesium Stearate | Lubricant | 0.5 | 5 |
| Total | | 100.00 | 1000 |

Seal, Intermediate and enteric coat formulations are calculated considering a surface area of 22.3 mm² and a batch size of in total 750g API mini tablets (diameter 2.5 mm concave).

### Seal Coating

At low to medium speed for 15 minutes until all components are dissolved. VIVAPHARM^{®} HPMC E3 and PEG 600 was added to a container with water and stirred gently stirring to prevent lumping. The solids content should be approximately 7.5. Spraying solution is passed through a 40mesh sieve and is gently stirred during the coating process.

**Table 18:Seal Coating**

| Material | Composition | Solid Composition Percentage | Weight per Tablet |
|---|---|---|---|
| VIVAPHARM^{®} HPMC E3 | 0.5mg/cm² | 90.9 | 0.22 mg |
| PEG 600 | 0.1 mg/cm² | 9.1 | 0.02 mg |
| Purified Water | On demand | | |
| | | | |
| Solid content | 7.5% w/w | | |
| Total solid weight gain | 0.6 mg/cm² | | |
| Total applied polymer | 0.5 mg/cm² | | |
| | | | |
| Mini Tablet weight uncoated | | | 10.7 mg |
| Mini Tablet weight seal coated | | | 10.9 mg |

### Intermediate Coating

VIVAPHARM^{®} HPMC E3 and lactose anhydrous was thoroughly dispersed in the 2/3rd portion of water while gently stirring to prevent lumping. In remained 1/3rd water PEG 600 and magnesium oxide light is homogenized at 2500rpm using a homogenizer (e. g. Ultra Turrax) for 10 minutes. The solids content should be approximately 15%. Pour the excipient suspension slowly into the VIVAPHARM^{®} HPMC E3 solution while stirring gently with a conventional stirrer. Pass the spray suspension through a 40mesh sieve. The spraying suspension was gently stirred during the coating process.

**Table 19:Intermediate Coating**

| **Material** | **Composition** | **Solid Composition Percentage** | **Weight per Tablet (mg)** |
|---|---|---|---|
| VIVAPHARM^{®} HPMC E3 | 4.mg/cm² | 44.78 | 0.99 |
| Magnesium Oxide Light | 4.mg/cm² | 44.78 | 0.99 |
| Lactose Anhydrous | 13.3 % on ds* | 6 | 0.13 |
| PEG 600 | 10% on ds* | 4.5 | 0.1 |
| Purified Water | On demand | | |
| | | | |
| Solid content | 15.0% w/w | | |
| Total solid weight gain | 9.9 mg/cm² | | |
| Total applied polymer | 4.0 mg/cm² | | |
| | | | |
| Mini Tablet weight uncoated | | | 10.87 |
| Mini Tablet weight intermediate coated | | | 13.08 mg |

| | | | |
|---|---|---|---|
| *Quantity based on dry polymer substance [%] | | | |

### Enteric Coating

The triethyl citrate and talc were homogenized in the water using a homogenizer (e. g. Ultra Turrax) for 10 minutes. Then the excipient suspension was poured slowly into the EUDRAGIT^{®} L 30 D-55 dispersion while stirring gently with a conventional stirrer and stirred for further 15 minutes. The final coating suspension was sieved throughout a 400µm sieve and stirred during the coating process.

**Table 20:Enteric Coating**

| **Material** | **Composition** | **Solid Composition Percentage** | **Weight per capsule** |
|---|---|---|---|
| EUDRAGIT^{®} L 30 D-55 | 3.7 mg/cm² | 62.50 | 0.82 mg |
| Triethyl citrate | 10.0 % on ds* | 6.25 | 0.08 mg |
| Talc | 50.0 % on ds* | 31.25 | 0.41 mg |
| Demineralized Water | On demand | n/a | |
| | | | |
| Solid content | 20.0% w/w | | |
| Total solid weight gain | 5.9 mg/cm² | 10.1 | |
| Total applied polymer | 3.7 mg/cm² | 6.3 | |
| | | | |
| Mini Tablet weight sub coated | | | 13.08 mg |
| Mini Tablet weight functional coated | | | 14.39 mg |

| | | | |
|---|---|---|---|
| *Quantity based on dry polymer substance [%] | | | |

### Mini-tablet Coating Process

### Process parameters for enteric coating of 750g of minitablets using GPCG-2 system

| | **EQUIPMENT SETUP:** | | | |
|---|---|---|---|---|
| Air distribution Plate | C | | | |
| Bottom Sieve | 250 # | | | |

| Finger bag | Bonnet | | | |
|---|---|---|---|---|
| **Variables** | **Unit** | **Seal Coating** | **Intermediate coating** | **Enteric coating** |
| Silicon tube ID/OD | mm | 3.2 | 3.2 | 3.2 |
| Filter shaking mode | - | Asynchronous | Asynchronous | Asynchronous |
| Filter shaking time | Sec | - | - | - |
| Filter shaking pause | Sec | - | - | - |
| Area temperature | °C | NMT 25 | NMT 25 | NMT 25 |
| Area Relative Humid | % | NMT 60 | NMT 60 | NMT 60 |
| Spray gun type | | Schlick | Schlick | Schlick |
| Spray gun number | | 1 | 1 | 1 |
| Nozzle bore | mm | 1 | 1 | 1 |
| Inlet temperature | °C | 45-55 | 35-45 | 30-35 |
| Product temperature | °C | 35-45 | 30-38 | 25-35 |
| Air flow | CFM | 55-70 | 60-80 | 70-90 |
| Column height | | 15- 25 | 15- 25 | 15-25 |
| Atomization pressure | Bar | 1.5-2.5 | 1.5-2.5 | 1.0-2.0 |
| Spray rate | g/min | 1.0-2.0 | 2.0 - 6.0 | 1.2-5.0 |

### Dissolution Test (according to USP 43 <711>)

### Dissolution system

| **Parameter** | **Condition** |
|---|---|
| Data System | Distek OptDiss 405 Version 3.1.2.0 Build 3637 |
| Instruments | CCD-DI-10027 |
| Dissolution Bath | Distek 2500 (SL No. 2501368) |
| Wavelength | 273 nm |
| Pathlength | 1 mm |
| Apparatus | USP Apparatus I (40 Mesh Basket) |
| Dissolution Bath Temperature | 37±0.5°C |
| Paddle speed | 75 rpm |
| 0.1N HCl | 500 mL |
| pH 3.0 HCl | 500 mL |
| PH 5 (Acetate Buffer) | 500 mL |
| Dissolution Sampling Points in 0.1N HCl | 60 and 120 minutes |
| Dissolution Sampling Points in pH 3.0 HCl | 135, 150 and 180 minutes |
| Dissolution Sampling Points in pH 5.0 AB | 135, 150 and 180 minutes |
| Infinity at 300 RPM | 255 minutes |

### Dissolution mediums

### 0.1N Hydrochloric acid (pH 1.2)

2. Mix 8.5 milliliters of concentrated hydrochloric acid in 1000 milliliters of de-ionized water and mix well.

### 0.001N Hydrochloric acid solution (pH 3.0)

3. Mix 1.0 milliliters of concentrated hydrochloric acid in 11.764 liters of de-ionized water and mix well.

4. If required, adjust it to pH 3.0 with 2. If required, adjust it to pH 3.0 with 0.1N HCl or 0.1N NaOH solution.

### Results

| **Dissolution testing in acid stage media** | |
|---|---|
| pH 1.2 (120 min) | Pass - less than 10% |

| **Dissolution testing (% drug release) in pH 3 media** | |
|---|---|
| pH 3.0 (15 min) | Pass - more than 80% (88%) |
| pH 3.0 (30 min) | Pass - more than 80% (100%) |
| pH 3.0 (60 min) | Pass - more than 80% (100%) |

### Dissolution mediums

### 0.1N Hydrochloric acid (pH 1.2)

1. Mix 8.5 milliliters of concentrated hydrochloric acid in 1000 milliliters of de-ionized water and mix well.

### Acetate Buffer solution (pH 5.0)

1. Weigh 5.08 g of Sodium Acetate Trihydrate to 500 mL of deionized water followed by 6.3 ml of 2N Glacial Acetic Acid and make up the volume to 1L.
2. If required, adjust it to pH 5.0 with 0.1N HCl or 0.1N NaOH solution.

### Results

| **Dissolution testing in acid stage media** | |
|---|---|
| pH 1.2 (120 min) | Pass - less than 10% |

| **Dissolution testing (% drug release) in pH 5 Acetate Buffer media** | |
|---|---|
| pH 5.0 (15 min) | Pass - more than 80% (85%) |
| pH 5.0 (30 min) | Pass - more than 80% (100%) |
| pH 5.0 (60 min) | Pass - more than 80% (100%) |

## Claims

1. Dosage form comprising
a) a core comprising at least one biologically active ingredient,
b) an intermediate coating layer (ICL) onto or above the core, comprising
i) at least one polymer is selected from celluloses, hydroxyethyl cellulose (HEC), hydroxypropyl cellulose (HPC), hydroxypropyl methyl cellulose (HPMC), hydroxyethyl methyl cellulose (HEMC), ethyl cellulose (EC), methyl cellulose (MC), cellulose esters, cellulose glycolates, polyethylene glycols, polyethylene oxides, polyvinyl pyrrolidone, polyvinyl acetate, polyvinyl alcohol, or a mixture thereof;
ii) at least one alkaline agent;
iii) at least one release acceleration agent selected from iron oxide, aluminium oxide, titanium dioxide, dimethyl sulfoxide, zinc oxide, sucrose, maltose, lactose, dextrates, glucose, fructose, dyes, or any mixture thereof;
iv) optionally at least one glidant;
v) optionally at least one plasticizer; and
vi) optionally at least one additive, different from i) to v); wherein
the at least one release acceleration agent is present in 0.1 to 20 wt.-% based on the weight of the at least one polymer; and
c) an enteric coating layer (ECL) onto or above the intermediate coating layer, comprising
i) at least one polymer is at least one (meth)acrylate copolymer;
ii) optionally at least one glidant;
iii) optionally at least one emulsifier;
iv) optionally at least one plasticizer;
v) optionally at least one biologically active ingredient; and
vi) optionally at least one additive, different from i) to v),
wherein the dosage form is a coated hard shell capsule, tablet or a coated mini tablet.

2. Dosage form according to claim 1, wherein the biologically active ingredient is a pharmaceutically active ingredient.

3. Dosage form according to any of the preceding claims, wherein the alkaline agent
i) is an alkali or an earth alkali metal salt; or
ii) is selected from calcium oxide, calcium carbonate, magnesium carbonate, magnesium oxide, sodium carbonate, sodium bicarbonate and sodium hydroxide or is any combination thereof; or
iii) is magnesium oxide or magnesium carbonate.

4. Dosage form according to any of the preceding claims, wherein the at least one release acceleration agent is Fe₂O₃, TiO₂, lactose, disodium 2-[[4-[ethyl-[(3-sulfonatophenyl)methyl]amino]phenyl]-[4-[ethyl-[(3-sulfonatophenyl)methyl]azaniumylidene]cyclohexa-2,5-dien-1-ylidene]methyl]benzenesulfonate or any mixture thereof.

5. Dosage form according to any of claims 1 to 4, wherein the at least one polymer in the intermediate coating layer and/or in the enteric coating layer, preferably enteric coating layer, is
i) a Core-Shell polymer, which is a copolymer obtained by a two stage emulsion polymerization process with a core with 70 to 80 % by weight, comprising polymerized units of 65 to 75 % by weight of ethyl acrylate and 25 to 35 % by weight of methyl methacrylate, and a shell with 20 to 30 % by weight, comprising polymerized units of 45 to 55 % by weight ethyl acrylate and 45 to 55 % by weight methacrylic acid; or
ii) an anionic polymer obtained by polymerizing 25 to 95 % by weight C1- to C12-alkyl esters of acrylic acid or of methacrylic acid and 75 to 5% by weight (meth)acrylate monomers with an anionic group; or
iii) a cationic (meth)acrylate copolymer obtained by polymerizing C1- to C4-alkyl esters of acrylic or of methacrylic acid and an alkyl ester of acrylic or of methacrylic acid with a tertiary or a quaternary ammonium group in the alkyl group; or
iv) a (meth)acrylate copolymer obtained by polymerizing methacrylic acid and ethyl acrylate, methacrylic acid and methyl methacrylate, ethyl acrylate and methyl methacrylate or methacrylic acid, methyl acrylate and methyl methacrylate; or
v) a (meth)acrylate copolymer obtained by polymerizing 40 to 60 % by weight of methacrylic acid and 60 to 40 % by weight of ethyl acrylate; or
vi) a (meth)acrylate copolymer obtained by polymerizing 60 to 80 % of ethyl acrylate and 40 to 20 % by weight of methyl methacrylate; or
vii) a (meth)acrylate copolymer obtained by polymerizing 5 to 15 % by weight methacrylic acid, 60 to 70 % by weight of methyl acrylate and 20 to 30 % by weight methyl methacrylate; or mixtures thereof.

6. Dosage form according to any of claims 1 to 4, wherein the at least one polymer in the intermediate coating layer and/or in the enteric coating layer, preferably enteric coating layer, is a mixture of
i) an anionic polymer having a Tgm ≥ 35°C, preferably 35 to 155 °C, more preferably 80 to 145 °C, most preferably 90 to 125 °C; and a second polymer is a polymer having a Tgm of ≤ 30 °C, preferably < 15 °C; or
ii) a (meth)acrylate copolymer obtained by copolymerizing 40 to 60 % by weight of methacrylic acid and 60 to 40 % by weight of ethyl acrylate and a (meth)acrylate copolymer obtained by polymerizing 60 to 80, preferably 60 to 78, % by weight of ethyl acrylate and 40 to 20, preferably 38 to 20, % by weight of methyl methacrylate preferably at a ratio from 20:1 to 1:20 by weight, and optionally up to 2 % by weight of (meth) acrylic acid; or
iii) a (meth)acrylate copolymer obtained by copolymerizing 5 to 15 % by weight methacrylic acid, 60 to 70 % by weight of methyl acrylate and 20 to 30 % by weight methyl methacrylate and a (meth)acrylate copolymer obtained by copolymerizing 40 to 60 % by weight of methacrylic acid and 60 to 40 % by weight of ethyl acrylate preferably at a ratio from 20:1 to 1:20 by weight.

7. Dosage form according to any of claims 1 to 4, wherein the at least one polymer in the intermediate coating layer and/or in the enteric coating layer is selected from at least one anionic cellulose, ethyl cellulose or starch comprising at least 35 % by weight amylose or mixtures thereof.

8. Dosage form according to any of claims 1 to 7, wherein the core comprises the biologically active ingredient
i) distributed in a matrix structure; or
ii) bound in a binder in a coating on a core.

9. Dosage form according to any of claims 1 to 8, wherein the core comprises the biologically active ingredient in the fill of a hard shell capsule, whereby the core is the hard shell capsule.

10. Method of obtaining the dosage form according to any of claims 1 to 9, wherein the intermediate coating layer is coated on the core via spray coating and thereafter the enteric coating layer is coated on the intermediate coating layer via spray coating.

11. Use of the dosage form according to any of the preceding claims for providing at least 80% drug release at pH value 5 within 60 min.

12. Use of the dosage form according to any of the preceding claims for providing at least 80% drug release at pH value 3 within 60 min.

## Patentansprüche

1. Darreichungsform, die Folgendes umfasst:
a) einen Kern, der mindestens einen biologischen Wirkstoff umfasst,
b) eine Zwischenüberzugsschicht (ICL) auf oder über dem Kern, die Folgendes umfasst:
i) mindestens ein Polymer, das aus Cellulosen, Hydroxyethylcellulose (HEC), Hydroxypropylcellulose (HPC), Hydroxypropylmethylcellulose (HPMC), Hydroxyethylmethylcellulose (HEMC), Ethylcellulose (EC), Methylcellulose (MC), Celluloseestern, Celluloseglycolaten, Polyethylenglycolen, Polyethylenoxiden, Polyvinylpyrrolidon, Polyvinylacetat, Polyvinylalkohol oder einer Mischung davon ausgewählt ist;
ii) mindestens ein alkalisches Mittel;
iii) mindestens ein Freisetzungsbeschleunigungsmittel, das aus Eisenoxid, Aluminiumoxid, Titandioxid, Dimethylsulfoxid, Zinkoxid, Saccharose, Maltose, Lactose, Dextraten, Glucose, Fructose, Farbstoffen oder einer beliebigen Mischung davon ausgewählt ist;
iv) gegebenenfalls mindestens ein Gleitmittel;
v) gegebenenfalls mindestens einen Weichmacher und
vi) gegebenenfalls mindestens ein von i) bis v) verschiedenes Additiv; wobei
das mindestens eine Freisetzungsbeschleunigungsmittel mit 0,1 bis 20 Gew.-%, bezogen auf das Gewicht des mindestens einen Polymers, vorhanden ist; und
c) eine magensaftresistente Überzugsschicht (ECL) auf oder über der Zwischenüberzugsschicht, die Folgendes umfasst:
i) mindestens ein Polymer, bei dem es sich um mindestens ein (Meth)Acrylat-Copolymer handelt;
ii) gegebenenfalls mindestens ein Gleitmittel;
iii) gegebenenfalls mindestens einen Emulgator;
iv) gegebenenfalls mindestens einen Weichmacher;
v) gegebenenfalls mindestens einen biologischen Wirkstoff und
vi) gegebenenfalls mindestens ein von i) bis v) verschiedenes Additiv,
wobei die Darreichungsform eine beschichtete Hartschalenkapsel, Tablette oder eine beschichtete Minitablette ist.

2. Darreichungsform nach Anspruch 1, wobei der biologische Wirkstoff ein pharmazeutischer Wirkstoff ist.

3. Darreichungsform nach einem der vorhergehenden Ansprüche, wobei das alkalische Mittel
i) ein Alkali- oder Erdalkalimetallsalz ist oder
ii) aus Calciumoxid, Calciumcarbonat, Magnesiumcarbonat, Magnesiumoxid, Natriumcarbonat, Natriumhydrogencarbonat und Natriumhydroxid ausgewählt oder eine beliebige Kombination davon ist oder
iii) Magnesiumoxid oder Magnesiumcarbonat ist.

4. Darreichungsform nach einem der vorhergehenden Ansprüche, wobei das mindestens eine Freisetzungsbeschleunigungsmittel Fe₂O₃, TiO₂, Lactose, Dinatrium-2-[[4-[ethyl-[(3-sulfonatophenyl)methyl]amino]phenyl]-[4-[ethyl-[(3-sulfonatophenyl)methyl]azaniumyliden]cyclohexa-2,5-dien-1-yliden]methyl]benzolsulfonat oder eine beliebige Mischung davon ist.

5. Darreichungsform nach einem der Ansprüche 1 bis 4, wobei das mindestens eine Polymer in der Zwischenüberzugsschicht und/oder in der magensaftresistenten Überzugsschicht, vorzugsweise in der magensaftresistenten Überzugsschicht, Folgendes ist:
i) ein Kern-Schale-Polymer, bei dem es sich um ein Copolymer handelt, das durch ein zweistufiges Emulsionspolymerisationsverfahren erhalten wird, mit einem Kern mit 70 bis 80 Gew.-%, der polymerisierte Einheiten aus 65 bis 75 Gew.-% Ethylacrylat und 25 bis 35 Gew.-% Methylmethacrylat umfasst, und einer Schale mit 20 bis 30 Gew.-%, die polymerisierte Einheiten aus 45 bis 55 Gew.-% Ethylacrylat und 45 bis 55 Gew.-% Methacrylsäure umfasst; oder
ii) ein anionisches Polymer, das erhalten wird, indem 25 bis 95 Gew.-% C1- bis C12-Alkylester von Acrylsäure oder Methacrylsäure und 75 bis 5 Gew.-% (Meth)Acrylsäure-Monomere mit einer anionischen Gruppe polymerisiert werden; oder
iii) ein kationisches (Meth)Acrylat-Copolymer, das erhalten wird, indem C1- bis C4-Alkylester von Acryl- oder Methacrylsäure und ein Alkylester von Acryl- oder Methacrylsäure mit einer tertiären oder einer quartären Ammoniumgruppe in der Alkylgruppe polymerisiert werden; oder
iv) ein (Meth)Acrylat-Copolymer, das erhalten wird, indem Methacrylsäure und Ethylacrylat, Methacrylsäure und Methylmethacrylat, Ethylacrylat und Methylmethacrylat oder Methacrylsäure, Methylacrylat und Methylmethacrylat polymerisiert werden; oder
v) ein (Meth)Acrylat-Copolymer, das erhalten wird, indem 40 bis 60 Gew.-% Methacrylsäure und 60 bis 40 Gew.- % Ethylacrylat polymerisiert werden; oder
vi) ein (Meth)Acrylat-Copolymer, das erhalten wird, indem 60 bis 80 Gew.-% Ethylacrylat und 40 bis 20 Gew.-% Methylmethacrylat polymerisiert werden; oder
vii) ein (Meth)Acrylat-Copolymer, das erhalten wird, indem 5 bis 15 Gew.-% Methacrylsäure, 60 bis 70 Gew.-% Methylacrylat und 20 bis 30 Gew.-% Methylmethacrylat polymerisiert werden; oder Mischungen davon.

6. Darreichungsform nach einem der Ansprüche 1 bis 4, wobei das mindestens eine Polymer in der Zwischenüberzugsschicht und/oder in der magensaftresistenten Überzugsschicht, vorzugsweise in der magensaftresistenten Überzugsschicht, eine Mischung von Folgendem ist:
i) einem anionischen Polymer mit einer Tgm ≥ 35 °C, vorzugsweise von 35 bis 155 °C, stärker bevorzugt von 80 bis 145 °C, am meisten bevorzugt von 90 bis 125 °C; und einem zweiten Polymer, bei dem es sich um ein Polymer mit einer Tgm ≤ 30 °C, vorzugsweise < 15 °C, handelt; oder
ii) einem (Meth)Acrylat-Copolymer, das erhalten, wird, indem 40 bis 60 Gew.-% Methacrylsäure und 60 bis 40 Gew.-% Ethylacrylat copolymerisiert werden, und einem (Meth)Acrylat-Copolymer, das erhalten, wird, indem 60 bis 80, vorzugsweise 60 bis 78 Gew.-% Ethylacrylat und 40 bis 20, vorzugsweise 38 bis 20 Gew.-% Methylmethacrylat, vorzugsweise in einem Verhältnis von 20:1 bis 1:20, bezogen auf das Gewicht, und gegebenenfalls bis zu 2 Gew.-% (Meth)Acrylsäure polymerisiert werden; oder
iii) einem (Meth)Acrylat-Copolymer, das erhalten wird, indem 5 bis 15 Gew.-% Methacrylsäure, 60 bis 70 Gew.-% Methylacrylat und 20 bis 30 Gew.-% Methylmethacrylat copolymerisiert werden, und einem (Meth)Acrylat-Copolymer, das erhalten wird, indem 40 bis 60 Gew.-% Methacrylsäure und 60 bis 40 Gew.-% Ethylacrylat, vorzugsweise in einem Verhältnis von 20:1 bis 1:20, bezogen auf das Gewicht, polymerisiert werden.

7. Darreichungsform nach einem der Ansprüche 1 bis 4, wobei das mindestens eine Polymer in der Zwischenüberzugsschicht und/oder in der magensaftresistenten Überzugsschicht aus anionischer Cellulose, Ethylcellulose und/oder mindestens 35 Gew.-% Amylose umfassender Stärke oder Mischungen davon ausgewählt ist.

8. Darreichungsform nach einem der Ansprüche 1 bis 7, wobei der Kern den biologischen Wirkstoff
i) verteilt in einer Matrixstruktur oder
ii) gebunden in einem Bindemittel in einem Überzug auf einem Kern umfasst.

9. Darreichungsform nach einem der Ansprüche 1 bis 8, wobei der Kern den biologischen Wirkstoff in der Füllung einer Hartschalenkapsel umfasst, wodurch der Kern die Hartschalenkapsel ist.

10. Verfahren zum Erhalt der Darreichungsform nach einem der Ansprüche 1 bis 9, wobei die Zwischenüberzugsschicht mittels Sprühbeschichten auf den Kern aufgetragen wird und danach die magensaftresistente Überzugsschicht mittels Sprühbeschichten auf die Zwischenüberzugsschicht aufgetragen wird.

11. Verwendung der Darreichungsform nach einem der vorhergehenden Ansprüche zur Bereitstellung einer Arzneimittelfreisetzung von mindestens 80 % bei einem pH-Wert von 5 innerhalb von 60 min.

12. Verwendung der Darreichungsform nach einem der vorhergehenden Ansprüche zur Bereitstellung einer Arzneimittelfreisetzung von mindestens 80 % bei einem pH-Wert von 3 innerhalb von 60 min.

## Revendications

1. Forme posologique comprenant
a) un noyau comprenant au moins un ingrédient biologiquement actif,
b) une couche d'enrobage intermédiaire (ICL) sur ou au-dessus du noyau, comprenant
i) au moins un polymère qui est choisi parmi les celluloses, l'hydroxyéthylcellulose (HEC), l'hydroxypropylcellulose (HPC), l'hydroxypropylméthylcellulose (HPMC), l'hydroxyéthylméthylcellulose (HEMC), l'éthylcellulose (EC), la méthylcellulose (MC), les esters de cellulose, les glycolates de cellulose, les polyéthylène glycols, les poly(oxyde d'éthylène), une polyvinylpyrrolidone, un poly(acétate de vinyle), un poly(alcool vinylique) ou un mélange de ceux-ci ;
ii) au moins un agent alcalin ;
iii) au moins un agent d'accélération de libération choisi parmi l'oxyde de fer, l'oxyde d'aluminium, le dioxyde de titane, le diméthylsulfoxyde, l'oxyde de zinc, le saccharose, le maltose, le lactose, les dextrates, le glucose, le fructose, les colorants ou un quelconque mélange de ceux-ci ;
iv) éventuellement au moins un agent glissant ;
v) éventuellement au moins un plastifiant ; et
vi) éventuellement au moins un additif, différent de i) à v) ; dans laquelle
l'agent ou les agents d'accélération de libération sont présents en une quantité de 0,1 à 20 % en poids par rapport au poids de l'au moins un polymère ; et
c) une couche d'enrobage entérique (ECL) sur ou au-dessus de la couche d'enrobage intermédiaire, comprenant
i) au moins un polymère qui est au moins un copolymère de (méth)acrylate ;
ii) éventuellement au moins un agent glissant ;
iii) éventuellement au moins un émulsifiant ;
iv) éventuellement au moins un plastifiant ;
v) éventuellement au moins un ingrédient biologiquement actif ; et
vi) éventuellement au moins un additif, différent de i) à v),
dans laquelle la forme posologique est une gélule à enveloppe dure enrobée, un comprimé ou un minicomprimé enrobé.

2. Forme posologique selon la revendication 1, dans laquelle l'ingrédient biologiquement actif est un ingrédient pharmaceutiquement actif.

3. Forme posologique selon l'une quelconque des revendications précédentes, dans laquelle l'agent alcalin
i) est un sel de métal alcalin ou de métal alcalino-terreux ; ou
ii) est choisi parmi l'oxyde de calcium, le carbonate de calcium, le carbonate de magnésium, l'oxyde de magnésium, le carbonate de sodium, le bicarbonate de sodium et l'hydroxyde de sodium ou une quelconque combinaison de ceux-ci ; ou
iii) est l'oxyde de magnésium ou le carbonate de magnésium.

4. Forme posologique selon l'une quelconque des revendications précédentes, dans laquelle l'au moins un agent d'accélération de libération est Fe₂O₃, TiO₂, le lactose, le 2-[[4-[éthyl-[(3-sulfonatophényl)méthyl]amino]phényl]-[4-[éthyl-[(3-sulfonatophényl)méthyl]azaniumylidène]cyclohexa-2,5-dién-1-ylidène]méthyl]benzènesulfonate disodique ou un quelconque mélange de ceux-ci.

5. Forme posologique selon l'une quelconque des revendications 1 à 4, dans laquelle l'au moins un polymère dans la couche d'enrobage intermédiaire et/ou dans la couche d'enrobage entérique, de préférence dans la couche d'enrobage entérique, est
i) un polymère noyau-enveloppe, qui est un copolymère obtenu par un procédé de polymérisation en émulsion en deux stades comportant un noyau de 70 à 80 % en poids, comprenant des unités polymérisées de 65 à 75 % en poids d'acrylate d'éthyle et de 25 à 35 % en poids de méthacrylate de méthyle, et une enveloppe de 20 à 30 % en poids, comprenant des unités polymérisées de 45 à 55 % en poids d'acrylate d'éthyle et de 45 à 55 % en poids d'acide méthacrylique ; ou
ii) un polymère anionique obtenu par polymérisation de 25 à 95 % en poids d'esters d'alkyle en C1 à C12 d'acide acrylique ou d'acide méthacrylique et de 75 à 5 % en poids de monomères de (méth)acrylate comportant un groupe anionique ; ou
iii) un copolymère de (méth)acrylate cationique obtenu par polymérisation d'esters d'alkyle en C1 a C4 d'acide acrylique ou d'acide méthacrylique et d'un ester d'alkyle d'acide acrylique ou d'acide méthacrylique comportant un groupe d'ammonium tertiaire ou un groupe d'ammonium quaternaire dans le groupe alkyle ; ou
iv) un copolymère de (méth)acrylate obtenu par polymérisation d'acide méthacrylique et d'acrylate d'éthyle, d'acide méthacrylique et de méthacrylate de méthyle, d'acrylate d'éthyle et de méthacrylate de méthyle ou d'acide méthacrylique, d'acrylate de méthyle et de méthacrylate de méthyle ; ou
v) un copolymère de (méth)acrylate obtenu par polymérisation de 40 à 60 % en poids d'acide méthacrylique et de 60 à 40 % en poids d'acrylate d'éthyle ; ou
vi) un copolymère de (méth)acrylate obtenu par polymérisation de 60 à 80 % d'acrylate d'éthyle et de 40 à 20 % en poids de méthacrylate de méthyle ; ou
vii) un copolymère de (méth)acrylate obtenu par polymérisation de 5 à 15 % en poids d'acide méthacrylique, de 60 à 70 % en poids d'acrylate de méthyle et de 20 à 30 % en poids de méthacrylate de méthyle ; ou des mélanges de ceux-ci.

6. Forme posologique selon l'une quelconque des revendications 1 à 4, dans laquelle l'au moins un polymère dans la couche d'enrobage intermédiaire et/ou dans la couche d'enrobage entérique, de préférence dans la couche d'enrobage entérique, est un mélange de
i) un polymère anionique ayant une Tgm ≥ 35 °C, de préférence 35 à 155 °C, plus préférablement 80 à 145 °C, le plus préférablement 90 à 125 °C ; et un second polymère qui est un polymère ayant une Tgm ≤ 30 °C, de préférence < 15 °C ; ou
ii) un copolymère de (méth)acrylate obtenu par copolymérisation de 40 à 60 % en poids d'acide méthacrylique et de 60 à 40 % en poids d'acrylate d'éthyle et un copolymère de (méth)acrylate obtenu par polymérisation de 60 à 80 %, de préférence de 60 à 78 % en poids d'acrylate d'éthyle et de 40 à 20 %, de préférence de 38 à 20 % en poids de méthacrylate de méthyle, de préférence en un rapport de 20:1 à 1:20 en poids, et éventuellement jusqu'à 2 % en poids d'acide (méth)acrylique ; ou
iii) un copolymère de (méth)acrylate obtenu par copolymérisation de 5 à 15 % en poids d'acide méthacrylique, de 60 à 70 % en poids d'acrylate de méthyle et de 20 à 30 % en poids de méthacrylate de méthyle et un copolymère de (méth)acrylate obtenu par copolymérisation de 40 à 60 % en poids d'acide méthacrylique et de 60 à 40 % en poids d'acrylate d'éthyle, de préférence en un rapport de 20:1 à 1:20 en poids.

7. Forme posologique selon l'une quelconque des revendications 1 à 4, dans laquelle l'au moins un polymère dans la couche d'enrobage intermédiaire et/ou dans la couche d'enrobage entérique est choisi parmi au moins une cellulose anionique, une éthylcellulose ou un amidon comprenant au moins 35 % en poids d'amylose ou des mélanges de ceux-ci.

8. Forme posologique selon l'une quelconque des revendications 1 à 7, dans laquelle le noyau comprend l'ingrédient biologiquement actif
i) distribué dans une structure matricielle ; ou
ii) lié dans un liant dans un enrobage sur un noyau.

9. Forme posologique selon l'une quelconque des revendications 1 à 8, dans laquelle le noyau comprend l'ingrédient biologiquement actif dans le remplissage d'une gélule à enveloppe dure, le noyau étant la gélule à enveloppe dure.

10. Procédé d'obtention de la forme posologique selon l'une quelconque des revendications 1 à 9, dans lequel la couche d'enrobage intermédiaire est enrobée sur le noyau par enrobage par pulvérisation et ensuite la couche d'enrobage entérique est enrobée sur la couche d'enrobage intermédiaire par enrobage par pulvérisation.

11. Utilisation de la forme posologique selon l'une quelconque des revendications précédentes pour fournir une libération de médicament d'au moins 80 % à une valeur de pH de 5 en 60 min.

12. Utilisation de la forme posologique selon l'une quelconque des revendications précédentes pour fournir une libération de médicament d'au moins 80 % à une valeur de pH de 3 en 60 min.
